# EUROPEAN PATENT APPLICATION

(11) **EP 4 434 966 A1**
(43) Date of publication of application: **25.09.2024**
(21) Application number: 22896139.7
(22) Date of filing: 18.11.2022
(51) Int. Cl.: C07D 207/36, A61K 31/40, A61K 31/4439, A61P 1/00, A61P 1/04, A61P 35/00, C07D 401/04, C07D 401/12, A23L 33/10

(54) **PYRROLE DERIVATIVE OR PHARMACEUTICALLY OR SITOLOGICALLY ACCEPTABLE SALT THEREOF, AND COMPOSITION FOR PREVENTION, AMELIORATION OR TREATMENT OF GASTROINTESTINAL DISORDERS COMPRISING SAME AS ACTIVE INGREDIENT**

(30) Priority: 19.11.2021 KR 20210160052; 20.06.2022 KR 20220075014
(71) Applicant: Hana Pharm. Co. Ltd., Hwaseong-si, Gyeonggi-do 18608 (KR)
(72) Inventor: YOO, Jae Ho, Hwaseong-si, Gyeonggi-do 18497 (KR); IM, Song I, Ansan-si, Gyeonggi-do 15521 (KR); LEE, Kyu Hang, Yongin-si, Gyeonggi-do 17083 (KR); KIM, Dong Hoon, Hwaseong-si, Gyeonggi-do 18617 (KR); OH, Eun Hye, Suwon-si, Gyeonggi-do 16454 (KR); LEE, Dong Yeoul, Hwaseong-si, Gyeonggi-do 18617 (KR); JUNG, Ki Moon, Hwaseong-si, Gyeonggi-do 18429 (KR); KWAK, Da Jin, Cheongju-si, Chungcheongbuk-do 28558 (KR); KIM, Seo Yeon, Seongnam-si, Gyeonggi-do 13531 (KR); KIM, Ye Rin, Gumi-si, Gyeongsangbuk-do 39459 (KR); MIN, Chang Ho, Cheongdo-gun, Gyeongsangbuk-do 38332 (KR)
(74) Representative: Dr. Schön, Neymeyr & Partner Patentanwälte mbB
(86) International application number: PCT/KR2022/018323
(87) International publication number: WO 2023/090942

(57) **Abstract**

The present invention relates to a pyrrole derivative or a pharmaceutically or sitologically acceptable salt thereof, and composition for prevention, amelioration or treatment of gastrointestinal disorders comprising same as active ingredient. Through the results, it can be confirmed that the pharmacokinetic parameters, oral bioavailability and storage stability of the compound of the present invention are remarkably improved due to deuteration of the terminal methylamine moiety. The pyrrole derivative or the pharmaceutically or sitologically acceptable salt thereof the present invention exhibits excellent anticancer activity in addition to excellent proton pump inhibitory activity, and has improved pharmacokinetic parameters and chemical stability, and thus can be effectively used in the prevention, amelioration or treatment of gastrointestinal disorders, including gastric cancer.

## Description

### TECHNICAL FIELD

The present invention relates to a pyrrole derivative or a pharmaceutically or sitologically acceptable salt thereof, and composition for prevention, amelioration or treatment of gastrointestinal disorders comprising same as active ingredient.

### BACKGROUND

Many gastrointestinal diseases commonly caused by modern people require control of gastric acid secretion. These gastrointestinal diseases comprise Zollinger/Ellison syndrome (ZES), gastroesophageal reflux disease (GERD), peptic ulcers such as gastric and duodenal ulcers, esophagitis and stomach cancer, but the present invention is not limited thereto. In particular, gastrointestinal diseases such as peptic ulcers may be accompanied by serious complications and are prevalent in industrialized countries.

Currently, the main therapeutic agents used to treat gastrointestinal diseases such as GERD and stomach ulcers comprise antacids that neutralize excess gastric secretions, anticholinergics that reduce acid secretion, histamine H2-receptor antagonists, which inhibit the release of gastric acid, and proton pump inhibitors (PPIs), which reduce gastric acid secretion. In particular, PPI acts by inhibiting the H+/K+-ATPase proton pump activity, which is the acid secretion mechanism of gastric cells. Since PPI was first introduced to the market in 1988 and has been clinically applied for decades, numerous PPI agents have been preferred as drugs for controlling gastric acid secretion and have been marketed. Representative PPI agents comprise omeprazole.

The above-described PPIs have a clear effect of inhibiting gastric acid secretion. However, they had the disadvantages of having to be taken 2 hours before a meal, being unstable in acidic conditions, and having limitations in pharmacokinetics and pharmacodynamics. These disadvantages have been overcome in a new gastric acid secretion inhibitor, a potassium-competitive gastric acid secretion inhibitor.

As a new type of acid inhibitors, potassium-competitive gastric acid secretion inhibitors (P-CABs) can inhibit the activity of H+/K+-ATPase by competitive binding of K+, and have a mechanism of action clearly different from those of PPIs such that P-CABs can be called gastric acid pump inhibitors. P-CABs have the properties of lipophilicity, slightly alkaline, high dissociation constant and stable at low pH values. Representative P-CABs comprise vonoprazan.

Although a series of potassium-competitive gastric acid secretion inhibitors (P-CABs) are currently available, there is still a need for the development of new compounds with novel drug effects.

For example, helicobacter pylori removal therapy may require combination administration with antibiotics and may need to be combined with drugs for hyperlipidemia or hypertension. Substituting hydrogen in a position that is metabolized by the liver with deuterium may result in increase of blood levels by decreased hepatic metabolism. The benefits of deuterium substitution are an increased convenience due to extended dosing intervals, reduced potential for drug interactions, and reduced side effects due to metabolites. A representative deuterium-substituted drug is TEVA's Austedo^{®}, which was approved by the FDA in 2017. [Drug Des Devel Ther. 2018; 12: 313-319]. In addition to Austedo, methods for substituting deuterium to improve metabolic stability and compound stability have been described in J. Med. Chem. 2019, 62, 11, 5276-5297. In addition, in order to improve the therapeutic effect of cancer and related diseases, a case of substituting deuterium [KR 10-2029135B1] and a case of substituting deuterium in vonoprazan to improve the efficacy and PK absorption [US10912769B2], etc. are introduced as research methods that can increase the metabolic stability of compounds and the stability of compounds.

The present inventors obtained novel compound with advanced the stability and PK absorption thereof, when the compound is substituted with Deuterium, and conducted research to prepare a composition having new drug effects in addition to gastric acid secretion inhibitory activity. As a result, the present inventors discovered that the pyrrole derivative represented by the following formula 1 has excellent anti-tumor activity in addition to excellent proton pump inhibitory activity, and improved pharmacokinetic parameters and chemical stability as compared to conventional PPI and P-CAB drugs, and thus have completed a composition for preventing, improving, or treating gastrointestinal diseases.

### DETAILED DESCRIPTION

### TECHNICAL PROBLEM

The object of the present invention is to provide a pyrrole derivative for preventing, improving or treating gastrointestinal diseases, and a method for producing same.

### SOLUTION TO PROBLEM

In order to achieve the above-described object of the present invention, the present invention provides a compound represented by the following formula 1: 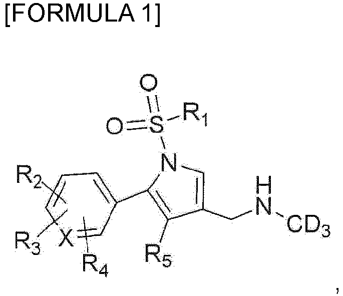 or a pharmaceutically or sitologically acceptable salt thereof,
wherein
R₁ is hydrogen, phenyl, quinolinyl, pyridinyl, pyrimidinyl, piperidinyl, thienyl or imidazolyl, wherein R₁ except hydrogen may be unsubstituted or substituted with 1 to 5 substituents independently selected from the group consisting of C₁-C₆ alkyl, C₁-C₆ alkoxy, C₁-C₄ haloalkyl, C₁-C₄ haloalkoxy, halogen, cyano, nitro, phenyl and halophenyl,
R₂, R₃ and R₄ are each independently hydrogen or halogen, provided that R₂, R₃ and R₄ cannot be hydrogen at the same time,
R₅ is hydrogen, methoxy, fluoro, chloro or hydroxy, and
X is carbon or nitrogen.

In addition, the present invention provides a pharmaceutical composition for preventing or treating gastrointestinal diseases, comprising the compound represented by formula 1, or a pharmaceutically acceptable salt thereof.

In addition, the present invention provides a pharmaceutical composition for preventing or treating gastrointestinal diseases, comprising the compound represented by formula 1, or a sitologically acceptable salt thereof.

In addition, the present invention provides a method for producing a pyrrole derivative comprising (1) reacting a compound of the following formula 2 and a sulfonyl compound in the presence of an organic solvent such as chloroform and sodium hydride as a base to prepare a compound of following formula 3; and (2) reacting the compound of the following formula 3 and deuterated methylamine (methyl-d₃-amine) in the presence of an organic solvent to prepare a compound of the following formula 4: 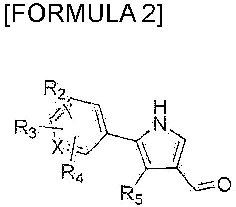 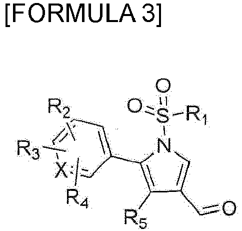 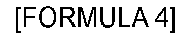 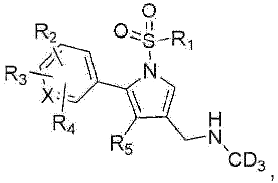 wherein
R₁ is hydrogen, phenyl, quinolinyl, pyridinyl, pyrimidinyl, piperidinyl, thienyl or imidazolyl, wherein R₁ except hydrogen may be unsubstituted or substituted with 1 to 5 substituents independently selected from the group consisting of C₁-C₆ alkyl, C₁-C₆ alkoxy, C₁-C₄ haloalkyl, C₁-C₄ haloalkoxy, halogen, cyano, nitro, phenyl and halophenyl,
R₂, R₃ and R₄ are each independently hydrogen or fluoro, provided that R₂, R₃ and R₄ cannot be hydrogen at the same time,
R₅ is hydrogen or chloro, and
X is carbon or nitrogen.

In addition, the present invention provides a method for producing a pyrrole derivative of formula 13, comprising (1) reacting a compound of the following formula 9 and a sulfonyl compound (R₁SO₂Cl) in the presence of a chloroform organic solvent and sodium hydride as a base to prepare a compound of the following formula 10; (2) reacting the compound of the following formula 10 and lithium aluminum hydride (LAH) as a reducing agent in the presence of an organic solvent to prepare a compound of the following formula 11; (3) reacting the compound of the following formula 11 in the presence of Dess-Martin periodinane as an oxidizing agent to prepare a compound of the following formula 12; and (4) reacting the compound of the following formula 12 and deuterated methylamine (methyl-d₃-amine) in the presence of an organic solvent to prepare a compound of the following formula 13: 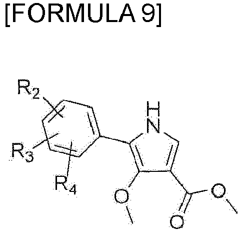 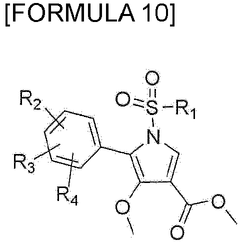 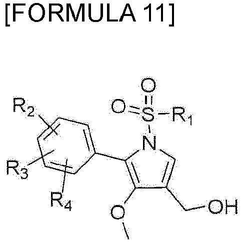 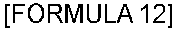 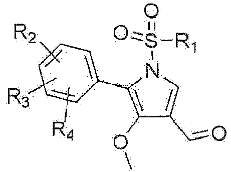 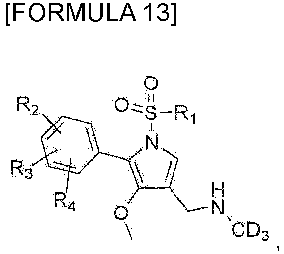 wherein
R₁ is hydrogen, phenyl, quinolinyl, pyridinyl, pyrimidinyl, piperidinyl, thienyl or imidazolyl, in which R₁ except hydrogen may be unsubstituted or substituted with 1 to 5 substituents independently selected from the group consisting of C₁-C₆ alkyl, C₁-C₆ alkoxy, C₁-C₄ haloalkyl, C₁-C₄ haloalkoxy, halogen, cyano, nitro, phenyl and halophenyl, and
R₂, R₃ and R₄ are each independently hydrogen or fluoro, provided that R₂, R₃ and R₄ cannot be hydrogen at the same time.

### ADVANTAGEOUS EFFECTS OF INVENTION

The pyrrole derivative, or pharmaceutically or sitologically acceptable salt thereof of the present invention has excellent proton pump inhibitory activity and anticancer activity, improved pharmacokinetic parameters such as half-life, improved bioavailability, and improved chemical stability so as to be used in preventing, improving or treating gastrointestinal diseases.

### BRIEF DESCRIPTION OF DRAWINGS

FIG. 1 is a graph showing the effect of compounds according to working examples and control drugs of the present invention of inhibiting the proliferation on human gastric cancer cells (AGS Cells).
FIG. 2 is a graph showing the anticancer efficacy of the compounds in xenograft mouse models according to Example 7 of the present invention.

### DEMONSTRATION FOR THE INVENTION

Hereinafter, the present invention will be described in detail.

While researching to develop a drug with excellent proton pump inhibitory activity, the present inventors confirmed that the pyrrole derivative compound represented by the following formula 1 has excellent proton pump inhibitory activity, effectively inhibits the proliferation of gastric cancer cells, improved pharmacokinetic and pharmacodynamic profiles in animal models, significantly increased oral bioavailability, and significantly improved storage stability. Accordingly, the present inventors have completed the present invention.

According to one aspect of the present invention, there is provided a pyrrole derivative represented by the following formula 1 and a pharmaceutically or sitologically acceptable salt thereof: 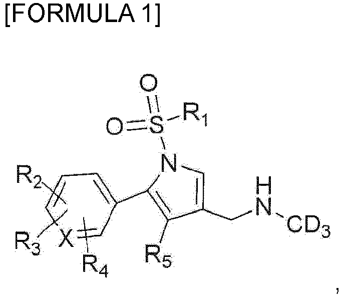 wherein
R₁ is hydrogen, phenyl, quinolinyl, pyridinyl, pyrimidinyl, piperidinyl, thienyl or imidazolyl, wherein R₁ except hydrogen may be unsubstituted or substituted with 1 to 5 substituents independently selected from the group consisting of C₁-C₆ alkyl, C₁-C₆ alkoxy, C₁-C₄ haloalkyl, C₁-C₄ haloalkoxy, halogen, cyano, nitro, phenyl and halophenyl,
R₂, R₃ and R₄ are each independently hydrogen or halogen, provided that R₂, R₃ and R₄ cannot be hydrogen at the same time,
R₅ is hydrogen, methoxy, fluoro, chloro or hydroxy, and
X is carbon or nitrogen..

In Formula 1 above, R₁ may preferably be phenyl, thienyl, or imidazolyl, more preferably, phenyl, thienyl, or imidazolyl, and, even more preferably, phenyl.

In addition, R₁ may preferably be unsubstituted or substituted with 1 to 3 substituents selected from the group consisting of methyl, trifluoromethyl, methoxy, trifluoromethoxy, fluoro, chloro, bromo, cyano and fluorophenyl. More preferably, it may be unsubstituted or substituted with one or two substituents selected from the group consisting of methyl, trifluoromethyl, methoxy, trifluoromethoxy, fluoro, chloro, bromo, cyano and fluorophenyl. More preferably, it may be one or two substituents selected from fluoro, chloro, and bromo. More preferably, it may be substituted with one fluoro substituent.

In addition, R₂, R₃ and R₄ may each independently be hydrogen or fluorine (however, R₂, R₃, and R₄ cannot be hydrogen at the same time). Most preferably, R₂ and R₃ are each hydrogen, and R₄ is fluoro.

In addition, R₅ may preferably be hydrogen, methoxy or chloro, and, more preferably, hydrogen.

In one embodiment, the compound may be represented by the following formula 1-1: 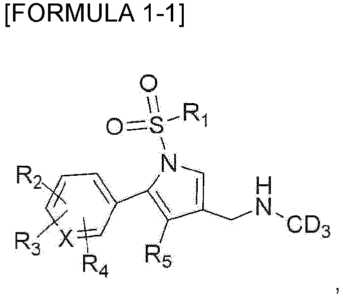 wherein
R₁ is hydrogen, phenyl, quinolinyl, pyridinyl, pyrimidinyl, piperidinyl, thienyl or imidazolyl, wherein R₁ except hydrogen may be unsubstituted or substituted with 1 to 5 substituents independently selected from the group consisting of C₁-C₆ alkyl, C₁-C₆ alkoxy, C₁-C₄ haloalkyl, C₁-C₄ haloalkoxy, halogen, cyano, nitro, phenyl and halophenyl,
R₂, R₃ and R₄ are each independently hydrogen or halogen, provided that R₂, R₃ and R₄ cannot be hydrogen at the same time,
R₅ is hydrogen, methoxy, fluoro, chloro or hydroxy, and
X is carbon or nitrogen.

Preferably, in the compound represented by formula 1-1 above, R₂ and R₃ may each independently be hydrogen or fluoro, and R₄ may be fluoro.

Representative examples of the compound represented by formula 1, or a pharmaceutically or sitologically acceptable salt thereof, are as follows:
1) N-((5-2-fluorophenyl)-1-((4-(trifluoromethyl)phenyl)sulfonyl)-1H-pyrrol-3-yl)methyl)methan-d₃-amine;
2) N-((5-(2-fluorophenyl)-1-tosyl-1H-pyrrol-3-yl)methyl)methan-d₃-amine;
3) N-((5-(2-fluorophenyl)-1-((4-methoxyphenyl)sulfonyl)-1H-pyrrol-3-yl)methyl)methan-d₃-amine;
4) 4-((2-(2-fluorophenyl)-4-(((methyl-d₃)amino)methyl)-1H-pyrrol-1-yl)sulfonyl)benzonitrile;
5) N-((1-((4-bromophenyl)sulfonyl)-5-(2-fluorophenyl)-1H-pyrrol-3-yl)methyl)methan-d₃-amine;
6) N-((1-((4-chlorophenyl)sulfonyl)-5-(2-fluorophenyl)-1H-pyrrol-3-yl)methyl)methan-d₃-amine;
7) N-((5-(2-fluorophenyl)-1-((4-fluorophenyl)sulfonyl)-1H-pyrrol-3-yl)methyl)methan-d₃-amine;
8) N-((5-(2-fluorophenyl)-1-((4-(trifluoromethoxy)phenyl)sulfonyl)-1H-pyrrol-3-yl)methyl)methan-d₃-amine;
9) N-((1-((4'-fluoro-[1,1'-biphenyl]-4-yl)sulfonyl)-5-(2-fluorophenyl)-1H-pyrrol-3-yl)methyl)methan-d₃-amine;
10) N-((1-((4-chlorophenyl)sulfonyl)-5-(3-fluorophenyl)-1H-pyrrol-3-yl)methyl)methan-d₃-amine;
11) N-((5-(3-fluorophenyl)-1-((4-methoxyphenyl)sulfonyl)-1H-pyrrol-3-yl)methyl)methan-d₃-amine;
12) N-((5-(2,4-difluorophenyl)-1-((4-fluorophenyl)sulfonyl)-1H-pyrrol-3-yl)methyl)methan-d₃-amine;
13) N-((1-((4-bromophenyl)sulfonyl)-5-(2,4-difluorophenyl)-1H-pyrrol-3-yl)methyl)methan-d₃-amine;
14) N-((5-(2,4-difluorophenyl)-1-((4-(trifluoromethyl)phenyl)sulfonyl)-1H-pyrrol-3-yl)methyl)methan-d₃-amine;
15) N-((5-(2,4-difluorophenyl)-1-((4-(trifluoromethoxy)phenyl)sulfonyl)-1H-pyrrol-3-yl)methyl)methan-d₃-amine;
16) N-((5-(2,4-difluorophenyl)-1-tosyl-1H-pyrrol-3-yl)methyl)methan-d₃-amine;
17) N-((5-(2,4-difluorophenyl)-1-((4-methoxyphenyl)sulfonyl)-1H-pyrrol-3-yl)methyl)methan-d₃-amine;
18) 4-((2-(2,4-difluorophenyl-4-(((methyl-d₃)amino)methyl)-1H-pyrrol-1-yl)sulfonyl)benzonitrile;
19) N-((5-(3-fluorophenyl)-1-((4-fluorophenyl)sulfonyl)-1H-pyrrol-3-yl)methyl)methan-d₃-amine;
20) N-((1-((4-bromophenyl)sulfonyl)-5-(3-fluorophenyl)-1H-pyrrol-3-yl)methyl)methan-d₃-amine;
21) N-((5-(3-fluorophenyl)-1-tosyl-1H-pyrrol-3-yl)methyl)methan-d₃-amine;
22) 4-((2-(3-fluorophenyl)-4-(((methyl-d₃)amino)methyl)-1H-pyrrol-1-yl)sulfonyl)benzonitrile;
23) N-((5-(3-fluorophenyl)-1-((4-(trifluoromethyl)phenyl)sulfonyl)-1H-pyrrol-3-yl)methyl)methan-d₃-amine;
24) N-((5-(4-fluorophenyl)-1-((4-fluorophenyl)sulfonyl)-1H-pyrrol-3-yl)methyl)methan-d₃-amine;
25) N-((1-((4-bromophenyl)sulfonyl)-5-(4-fluorophenyl)-1H-pyrrol-3-yl)methyl)methan-d₃-amine;
26) N-((1-((4-chlorophenyl)sulfonyl)-5-(4-fluorophenyl)-1H-pyrrol-3-yl)methyl)methan-d₃-amine;
27) N-((5-(4-fluorophenyl)-1-((4-methoxyphenyl)sulfonyl)-1H-pyrrol-3-yl)methyl)methan-d₃-amine;
28) N-((5-(4-fluorophenyl)-1-tosyl-1H-pyrrol-3-yl)methyl)methan-d₃-amine;
29) N-((5-(4-fluorophenyl)-1-((4-(trifluoromethoxy)phenyl)sulfonyl)-1H-pyrrol-3-yl)methyl)methan-d₃-amine;
30) N-((5-(4-fluorophenyl)-1-((4-(trifluoromethyl)phenyl)sulfonyl)-1H-pyrrol-3-yl)methyl)methan-d₃-amine;
31) 4-((2-(4-fluorophenyl)-4-(((methyl-d₃)amino)methyl)-1H-pyrrol-1-yl)sulfonyl)benzonitrile;
32) N-((5-(3-fluorophenyl)-1-((4-(trifluoromethoxy)phenyl)sulfonyl)-1H-pyrrol-3-yl)methyl)methan-d₃-amine;
33) N-((1-((4-fluorophenyl)sulfonyl)-5-(2-fluoropyridin-3-yl)-1H-pyrrol-3-yl)methyl)methan-d₃-amine;
34) N-((1-((4-chlorophenyl)sulfonyl)-5-(2-fluoropyridin-3-yl)-1H-pyrrol-3-yl)methyl)methan-d₃-amine;
35) N-((5-(2,4-difluorophenyl)-4-methoxy-1-(pyridin-3-ylsulfonyl)-1H-pyrrol-3-yl)methyl)methan-d₃-amine;
36) N-((5-(2-fluorophenyl)-4-methoxy-1-(pyridin-3-ylsulfonyl)-1H-pyrrol-3-yl)methyl)methan-d₃-amine;
37) N-((5-(2,4-difluorophenyl)-4-methoxy-1-((6-methoxypyridin-3-yl)sulfonyl)-1H-pyrrol-3-yl)methyl)methan-d₃-amine;
38) N-((4-methoxy-1-((6-(trifluoromethyl)pyridin-3-yl)sulfonyl)-5-(2,4,6-trifluorophenyl)-1H-pyrrol-3-yl)methyl)methan-d₃-amine;
39) N-((4-methoxy-1-(pyridin-3-ylsulfonyl)-5-(2,4,6-trifluorophenyl)-1H-pyrrol-3-yl)methyl)methan-d₃-amine; and
40) N-((4-methoxy-1-((6-methoxypyridin-3-yl)sulfonyl)-5-(2,4,6-trifluorophenyl)-1H-pyrrol-3-yl)methyl)methan-d₃-amine.

Preferred examples of the compound represented by formula 1, or a pharmaceutically or sitologically acceptable salt thereof in terms of proton pump inhibitory activity and antitumor activity are as follows:
2) N-((5-(2-fluorophenyl)-1-tosyl-1H-pyrrol-3-yl)methyl)methan-d₃-amine;
3) N-((5-(2-fluorophenyl)-1-((4-methoxyphenyl)sulfonyl)-1H-pyrrol-3-yl)methyl)methan-d₃-amine;
4) 4-((2-(2-fluorophenyl)-4-(((methyl-d₃)amino)methyl)-1H-pyrrol-1-yl)sulfonyl)benzonitrile;
7) N-((5-(2-fluorophenyl)-1-((4-fluorophenyl)sulfonyl)-1H-pyrrol-3-yl)methyl)methan-d₃-amine; and
35) N-((5-(2,4-difluorophenyl)-4-methoxy-1-(pyridin-3-ylsulfonyl)-1H-pyrrol-3-yl)methyl)methan-d₃-amine.

The compound represented by formula 1 or a salt thereof may have a substituent containing an asymmetric atom. In this case, the compound of formula 1 or a salt thereof may exist as an optical isomer such as (R), (S) and racemic form (RS). Therefore, unless otherwise indicated, the compound of formula 1 or a salt thereof comprises all optical isomers such as (R), (S) and racemic form (RS).

The compound represented by formula 1 may be in the form of a pharmaceutically or sitologically acceptable salt. The salt comprises common acid addition salts, for example, a salt derived from inorganic acids (e.g., hydrochloric acid, hydrobromic acid, sulfuric acid, sulfamic acid, phosphoric acid and nitric acid), and a salt derived from organic acids (e.g., acetic acid, propionic acid, succinic acid, glycolic acid, stearic acid, maleic acid, hydroxymaleic acid, phenylacetic acid, glutamic acid, benzoic acid, salicylic acid, sulfanilic acid, 2-acetoxy-benzoic acid, fumaric acid, toluenesulfonic acid, methanedisulfonic acid, ethanedisulfonic acid, oxalic acid, or trifluoroacetic acid). Preferably, the salt may be hydrochloride or fumarate.

For example, the compound represented by formula 1 may be prepared by any one method selected from Schemes 1 to 3 below.

In Schemes 1 to 3 below, each reaction time may be 15 minutes to 24 hours, preferably 30 minutes to 16 hours. In addition, the reaction temperature may be about -78°C to 120°C, preferably 0°C to 110°C, and more preferably 0°C to 70°C.

At this time, the reaction solvent (organic solvent) may be any one or a mixed solvent of two or more selected from alcohol (methanol, ethanol, butanol, isopropyl alcohol), ethers (ethyl propyl ether, methyl-tert-butyl ether, n-butyl ether, anisole, phenetol, cyclohexyl methyl ether, dimethyl ether, diethyl ether, dipropyl ether, diisopropyl ether, di-n-butyl ethers, diisobutyl ether, diisoamyl ether, ethylene glycol dimethyl ether, tetrahydrofuran, 1,4-dioxane, dichlorodiethyl ether and polyethers of ethylene oxide and/or propylene oxide), halohydrocarbons (tetrachloroethylene, tetrachloroethane, dichloropropane, methylene chloride, dichlorobutane, chloroform, tetrachloromethane, trichloroethane, trichloroethylene, pentachloroethane, difluorobenzene, 1,2-dichloroethane, chlorobenzene, bromobenzene, dichlorobenzene, chlorotoluene, trichlorobenzene), Nitrohydrocarbons (nitromethane, nitroethane, nitropropane, nitrobenzene, chloronitrobenzene, o-nitrotoluene), aromatic hydrocarbons (pentane, hexane, heptane, octane, cyclohexane, methylcyclohexane, petroleum ether, ligroin, octane, benzene, toluene, chlorobenzene, bromobenzene, nitrobenzene, xylene), aliphatic hydrocarbons, cycloaliphatic hydrocarbons, nitriles (acetonitrile, propionitrile, butyronitrile, isobutyronitrile, benzonitrile, m-chlorobenzonitrile), sulfones (dimethyl sulfone, diethyl sulfone, dipropyl sulfone, dibutyl sulfone, diphenyl sulfone, dihexyl sulfone, methylethyl sulfone, ethylpropyl sulfone, ethyl isobutyl sulfone and pentamethylene sulfone), sulfoxides (tetrahydrothiophene dioxide, dimethyl sulfoxide, tetramethylene sulfoxide, dipropyl sulfoxide, benzylmethyl sulfoxide, diisobutyl sulfoxide, dibutyl sulfoxide, diisoamyl sulfoxide), amines (trimethylamine, triethylamine, tripropylamine, tributylamine, n-methylmorpholine, pyridine and tetramethylenediamine), amides (hexamethylenephosphorictriamide, formamide, N-methylformamide, N,N-dimethylformamide, N,N-dipropylformamide, N,N-dibutylformamide, N-methylpyrrolidine , N-methylcaprolactam, 1,3-dimethyl-3,4,5,6-tetrahydro-2(1H)-pyrimidine, octylpyrrolidone, octylcaprolactam, 1,3-dimethyl-2-imide Dazolinedione, N-formylpiperidine, N,N'-1,4-diformylpiparazine), ketones (acetone, acetophenone, methyl ethyl ketone, methyl butyl ketone, methyl isobutyl ketone), esters (methyl acetate, ethyl acetate, butyl acetate, isobutyl acetate, dimethyl carbonate, dibutyl carbonate, and ethylene carbonate). Preferably, it may be one or more organic solvent selected from methanol, chloroform, tetrahydrofuran, and N,N-dimethylformamide.

In addition, the base for the reaction may be, but not particularly limited to, organic bases (e.g., N,N-dimethylaminopyridine (DMAP), pyridine, triethylamine, N,N-diisopropylethylamine and 1,8-diazabicyclo[5.4.0]undec-7-ene (DBU)), or inorganic bases (e.g., sodium carbonate, sodium bicarbonate, sodium hydroxide, lithium hydroxide, potassium hydroxide, barium hydroxide, sodium hydride (NaH), and potassium carbonate), which are used alone or in combination, in equivalent or excessive amounts.

In addition, the oxidizing agent for the reaction may be one selected from a compound, a peroxide and an N-haloimide compound comprising at least one heavy metal or a salt of a heavy metal. Preferably, it may be one selected from a compound containing at least one heavy metal or a salt of a heavy metal, and a peroxide. The compound and peroxide comprising at least one heavy metal or salt of a heavy metal mentioned above may specifically be iodine compounds, chlorides, chromium compounds, manganese dioxide, permanganic acid and perborates. More specifically, they may be Des-Martin periodinane, oxalyl chloride, chromate anhydride, chromium trioxide, potassium dichromate, sodium dichromate, potassium chromate, silver chromate, lead chromate, barium chromate, pyridinium chlorochromate, pyridinium dichromate, manganese dioxide, potassium permanganate, sodium permanganate, barium permanganate, lithium permanganate, sodium perborate.

In the following formulae 1 to 3, R₁ may be hydrogen, phenyl, quinolinyl, pyridinyl, pyrimidinyl, piperidinyl, thienyl or imidazolyl, wherein R₁ except hydrogen may be unsubstituted or substituted with 1 to 5 substituents independently selected from the group consisting of C₁-C₆ alkyl, C₁-C₆ alkoxy, C₁-C₄ haloalkyl, C₁-C₄ haloalkoxy, halogen, cyano, nitro, phenyl and halophenyl; R₂, R₃ and R₄ may each independently be hydrogen or fluoro, provided that R₂, R₃ and R₄ cannot be hydrogen at the same time; R₅ may be hydrogen or chloro; and X may be hydrogen, carbon or nitrogen.

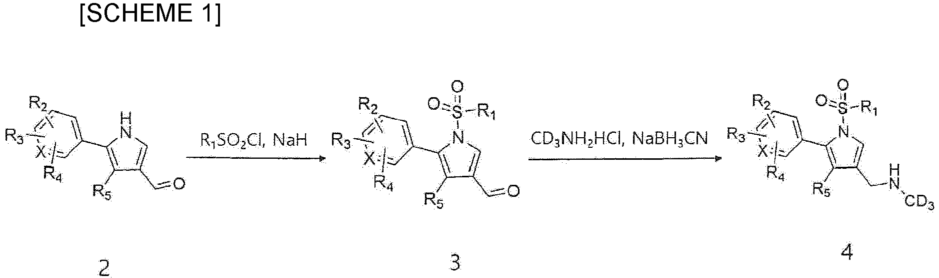

In Scheme 1, Step 1 is a step of preparing the compound represented by formula 2 into the compound represented by formula 3, which is a reaction of introducing a sulfonating group by using a sulfonyl compound (R₁SO₂Cl) and organic solvents such as chloroform and tetrahydrofuran in the presence of sodium hydride (NaH) as a base.

Step 2 is a reductive amination reaction, which reacts the compound represented by formula 3 with deuterated methylamine (methyl-d₃-amine) to convert same into the compound represented by formula 4. The reaction may be performed in the presence of a reducing agent such as Sodium cyanoborohydride and an organic solvent such as methanol and tetrahydrofuran.

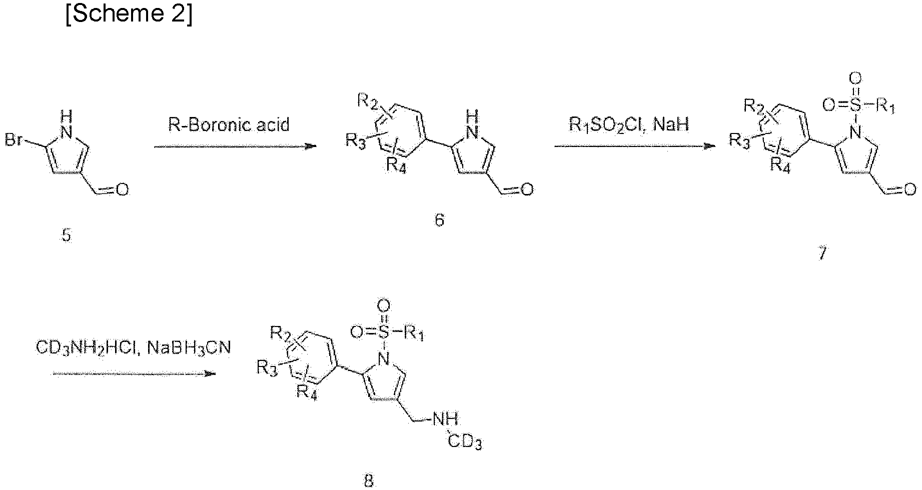

In Scheme 2, Step 1 is a step of preparing the compound represented by formula 5 and R-Boronic acid into the compound represented by formula 6 under a metal catalyst, which is also called Suzuki Coupling. The reaction may be performed in the presence of a base such as potassium carbonate and cesium carbonate and an organic solvent such as N,N-dimethylformamide.

Step 2 is a step of preparing the compound represented by formula 6 into the compound represented by formula 7, which is a reaction of introducing a sulfonating group by using a sulfonyl compound (R₁SO₂Cl), organic solvents such as chloroform and tetrahydrofuran in the presence of sodium hydride as a base.

Step 3 is a reductive amination reaction, which reacts the compound represented by formula 7 with deuterated methylamine (methyl-d₃-amine) to convert same into the compound represented by formula 8. The reaction may be performed in the presence of a reducing agent such as Sodium cyanoborohydride and an organic solvent such as methanol and tetrahydrofuran.

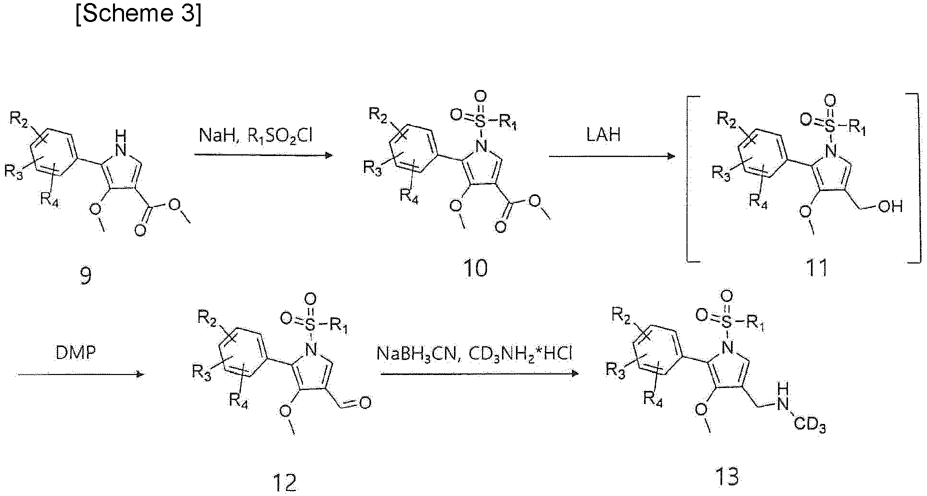

In Scheme 3, Step 1 is a step of preparing the compound represented by formula 9 into the compound represented by formula 10 under a metal catalyst, which is a reaction of introducing a sulfonating group by using a sulfonyl compound (R₁SO₂Cl) in the presence of an organic solvent such as chloroform and sodium hydride (NaH) as a base.

Step 2 is a step of converting the compound represented by formula 10 to the compound represented by formula 11 by using lithium aluminum hydride (LAH) as a reducing agent in the presence of an organic solvent such as tetrahydrofuran.

Step 3 is an oxidation reaction which converts the compound represented by formula 11 to the aldehyde compound of formula 12 by using Dess-Martin periodinane as an oxidizing agent. The reaction may be performed in the presence of an organic solvent such as methylene chloride and chloroform.

Step 4 is a reductive amination reaction, which reacts the compound represented by formula 12 with deuterated methylamine (methyl-d₃-amine) to convert same into the compound represented by formula 13. The reaction may be performed in the presence of a reducing agent such as Sodium cyanoborohydride and an organic solvent such as methanol and tetrahydrofuran.

In addition, the present invention provides a pharmaceutical composition for preventing or treating gastrointestinal diseases, comprising the compound represented by formula 1 or a pharmaceutically acceptable salt thereof.

In addition, the present invention provides a method for preventing, improving, or treating gastrointestinal diseases, comprising administering to a subject a composition comprising the compound represented by formula 1 or a pharmaceutically acceptable salt thereof.

In addition, the present invention provides a use in therapy of the compound represented by formula 1 or a pharmaceutically acceptable salt thereof.

The therapeutic use may be the treatment of gastrointestinal diseases.

The gastrointestinal disease may be one or more types selected from gastric ulcer, duodenal ulcer, gastritis, reflux esophagitis, gastric mucosal damage and stomach cancer, and preferably may be one or more types of peptic ulcer selected from gastric ulcer and duodenal ulcer.

In addition, the gastrointestinal disease may be a disease caused by excessive secretion of gastric acid.

As used herein, the term "prevention" refers to all actions that suppress or delay the onset of gastric ulcer, duodenal ulcer, gastritis, reflux esophagitis, gastric mucosal damage, and gastric cancer by administering the pharmaceutical composition of the present invention to an individual, and the term "treatment" refers to all actions that improve or benefit symptoms of gastric ulcer, duodenal ulcer, gastritis, reflux esophagitis, gastric mucosal damage, and gastric cancer by administering the pharmaceutical composition of the present invention to an individual.

The pharmaceutical composition according to the present invention may further comprise a pharmaceutically acceptable carrier, excipient or diluent.

As used herein, the term "pharmaceutically acceptable" refers to a compound commonly used in the pharmaceutical field that does not irritate the organism upon administration and does not inhibit the biological activity and properties of the administered compound.

As used herein, the term "comprising as an active ingredient" refers to comprising an amount sufficient to achieve the efficacy or activity of the compound of the present invention of preventing, improving or treating gastrointestinal diseases.

In the present invention, the type of the carrier is not particularly limited, and any carrier commonly used in the art can be used. Non-limiting examples of such carriers may comprise saline solution, sterile water, Ringer's solution, buffered saline solution, albumin injection solution, lactose, dextrose, sucrose, sorbitol, mannitol, xylitol, erythritol, maltitol, maltodextrin, glycerol and ethanol. These may be used alone or in combination of two or more types.

In addition, the pharmaceutical composition of the present invention may be used in addition with other pharmaceutically acceptable additives such as excipients, diluents, antioxidants, buffers and bacteriostatic agents, if necessary, and may further be used in addition with fillers, diluents, wetting agents, disintegrants, dispersant, surfactants, binders or lubricants.

In addition, the pharmaceutical composition of the present invention may be formulated and used in various dosage forms suitable for oral administration or non-oral administration.

Non-limiting examples of the formulations for oral administration may comprise troches, lozenges, tablets, aqueous suspensions, oily suspensions, formulated powders, granules, emulsions, hard capsules, soft capsules, syrup, or elixir.

In order to prepare the pharmaceutical composition of the present invention for oral administration, binding agents (e.g., lactose, saccharose, sorbitol, mannitol, starch, amylopectin, cellulose and gelatin); excipients (e.g., dicalcium phosphate); disintegrants (e.g., corn starch and sweet potato starch); and lubricants (e.g., magnesium stearate, calcium stearate, sodium stearyl fumarate and polyethylene glycol wax) may be used, and sweeteners, aromatics and syrups may also be used. Furthermore, in the case of capsules, in addition to the above-mentioned substances, a liquid carrier such as fatty oil may be additionally used.

Non-limiting examples of the non-oral preparations may comprise injection solutions, suppositories, powders for respiratory inhalation, aerosol preparations for sprays, ointments, powders for application, oils and creams.

In order to formulate the pharmaceutical composition of the present invention for non-oral administration, sterilized aqueous solutions, non-aqueous solutions, suspensions, emulsions, freeze-dried preparations and external preparations may be used. The non-aqueous solvent and suspension may comprise propylene glycol, polyethylene glycol, vegetable oil such as olive oil, and injectable ester such as ethyl oleate.

In addition, more specifically, when the pharmaceutical composition of the present invention is formulated as an injection solution, the composition of the present invention may be mixed in water with a stabilizer or buffer to prepare a solution or suspension, and may be formulated for unit administration in ampoules or vials. In addition, when the pharmaceutical composition of the present invention is formulated as an aerosol, a propellant may be added together with additives to disperse the water-dispersed concentrate or wet powder.

In addition, when the pharmaceutical composition of the present invention is formulated into an ointment, cream, etc., the pharmaceutical composition of the present invention may be formulated by using as carriers animal oil, vegetable oil, wax, paraffin, starch, tracant, cellulose derivatives, polyethylene glycol, silicone, bentonite, silica, talc and zinc oxide.

The pharmaceutically effective amount and effective dosage of the pharmaceutical composition of the present invention may vary depending on the formulation method, administration method, administration time, and/or administration route of the pharmaceutical composition, and may vary depending on several factors (e.g., type and degree of response to be achieved by administration of the pharmaceutical composition, type, age, weight, general health, symptoms or severity of disease, gender, diet and excretion of subject to be administered, drugs and other composition ingredients that are used in the subject simultaneously or in different times) and similar factors well known in the medical field. A person skilled in the art can easily determine and prescribe an effective dosage for a desired treatment.

The dosage for achieving more preferred effects of the pharmaceutical composition of the present invention is 0.01 to 100 mg/kg per day, more preferably 0.01 to 50 mg/kg. At this time, the concentration of the pyrrole derivative represented by formula 1 or a pharmaceutically acceptable salt thereof in the pharmaceutical composition is 0.01 to 20 mg/ml, more preferably 0.01 to 10 mg/ml. The pharmaceutical composition of the present invention may be administered once a day or divided into several times. At this time, if the content of the pyrrole derivative compound is less than the lower limit, the cell survival rate may be excellent, but the effect of improving or treating gastrointestinal diseases may not be achieved to a desired extent. Conversely, if the content exceeds the upper limit, the effect of improving or treating gastrointestinal diseases may not increase as the concentration increases, or the composition may be toxic.

The total effective amount of the pharmaceutical composition of the present invention may be administered to a patient as a single dose, or may be administered by a fractionated treatment protocol in which multiple doses are administered over a long period of time. The content of the active ingredient in the pharmaceutical composition of the present invention may vary depending on the severity of the disease.

The route and method of administration of the pharmaceutical composition of the present invention may be independent, and are not particularly limited. Any administration route and method may be used as long as the pharmaceutical composition can reach the desired area. The pharmaceutical composition may be administered by oral administration or non-oral administration.

The non--oral administration method may be, for example, intraperitoneal injection, intrarectal injection, subcutaneous injection, intravenous injection, intramuscular injection, intrauterine intrathecal injection, intracerebrovascular injection, or intrathoracic injection. Such method may also be the method of applying the composition to the diseased area, or spraying or inhaling same, but the present invention is not limited to thereto.

The pharmaceutical composition of the present invention may preferably be administered orally or by injection, and more preferably may be administered orally.

In addition, the pharmaceutical composition may be used alone or in combination with surgery, radiation therapy, hormone therapy, chemotherapy, and methods using biological response regulators.

In addition, the subject of administration of the pharmaceutical composition of the present invention may be a human being or an animal.

In addition, the present invention provides a health functional food composition for preventing or improving gastrointestinal diseases, comprising the compound represented by formula 1 or a sitologically acceptable salt thereof.

The gastrointestinal disease may be one or more types selected from gastric ulcer, duodenal ulcer, gastritis, reflux esophagitis, gastric mucosal damage and gastric cancer.

As used herein, the term "preventing" refers to anything that suppresses or delays the above gastrointestinal disease.

As used herein, the term "improving" refers to any action that reduces at least the severity of a parameter related to the condition being treated, for example, the severity of symptoms.

As used herein, the term "health functional food composition" refers to a food manufactured (comprising processing) by using raw materials or ingredients with functionality useful to the human body in accordance with legal standards (Article 3, Paragraph 1 of the Health Functional Food Act). The "health functional food composition" may differ in terminology or scope depending on the country, but may fall under the 'Dietary Supplement' in the United States, 'Food Supplement' in Europe, and Japan. 'Health functional food' or 'Food for Special Health Use (FOSHU)' in Japan, and 'health food' in China.

The health functional food composition may additionally comprise food additives, and suitability as a food additive is determined by the specifications and standards for the item in accordance with the general provisions and general test methods of the 'Food Additive Code,' unless otherwise specified.

The health functional food refers to a food prepared by adding the pyrrole derivative compound of the present invention to various food materials or by encapsulating, powdering and suspending, etc., which brings about a specific health effect when consumed. However, unlike drugs, the health functional food has the advantage of being made from food and not having any side effects that may occur when taking the drug for a long time. The health functional food composition of the present invention obtained in this way is very useful because it can be consumed on a daily basis. The amount of the pyrrole derivative compound added to such health functional food may vary depending on the type of health functional food being targeted and cannot be uniformly specified. However, the amount may be within a range that does not damage the original taste of the food, and is usually in the range of 0.001 to 50% by weight, preferably 0.01 to 10% by weight, relative to the target food. In addition, in the case of health functional foods in the form of pills, granules, tablets or capsules, the pyrrole derivative compound may be added in the range of usually 0.1 to 100% by weight, preferably 0.5 to 50% by weight. In one embodiment, the health functional food of the present invention may be in the form of powder, granules, tablets, capsules or beverages.

In addition, the health functional food may be manufactured in various forms according to conventional methods known in the art. General foods may be prepared by adding the pyrrole derivative compound to beverages (e.g., alcoholic beverages), fruits and processed foods thereof (e.g., canned fruit, bottled food, jam and mamalade), fish, meat and processed foods thereof (e.g., ham and sausages and corned beef), bread and noodles (e.g., udon, buckwheat noodles, ramen, spaghetti and macaroni), fruit juice, various drinks, cookies, taffy, dairy products (e.g., butter and cheese), edible vegetable oil, margarine, vegetable proteins, retort foods, frozen foods, and various seasonings (e.g., soybean paste, soy sauce and sauce), but the present invention is not limited thereto. In addition, nutritional supplements may be prepared by adding the pyrrole derivative compound to capsules, tablets, pills, etc., but the present invention is not limited thereto. In addition, the health functional food may be, for example, prepared in the form of tea, juice and drinks (health drinks) by liquefying the pyrrole derivative compound *per se*, or digested and consumed by granulating, encapsulating or powdering same, but the present invention is not limited thereto. In addition, in order to use the pyrrole derivative compound in the form of a food additive, it may be prepared as a powder, or extracted and used in the form of a concentrate.

When the health functional food composition for preventing or improving gastrointestinal diseases of the present invention is used as a health drink composition, the health drink composition may contain various flavoring agents or natural carbohydrates as additional ingredients like ordinary drinks. The natural carbohydrates described above may be monosaccharides such as glucose and fructose; disaccharides such as maltose and sucrose; polysaccharides such as dextrins and cyclodextrins; and sugar alcohols such as xylitol, sorbitol, and erythritol. Sweeteners may comprise natural sweeteners such as thaumatin and stevia extract; and synthetic sweeteners such as saccharin and aspartame. The proportion of natural carbohydrates is generally about 0.01 to 0.04 g, and preferably about 0.02 to 0.03 g per 100 mL of the composition of the present invention.

The pyrrole derivative compound of the present invention may be contained as an active ingredient in the health functional food composition, and the amount may be an amount effective to achieve the effect of prevention, alleviation or treatment of gastrointestinal diseases.

The dosage for achieving a more preferred effect of the health functional food composition is 0.01 to 100 mg/kg, more preferably 0.01 to 50 mg/kg. At this time, the concentration of the pyrrole derivative represented by formula 1 or a sitologically acceptable salt thereof in the health functional food composition is 0.01 to 20 mg/ml, more preferably 0.01 to 10 mg/ml. At this time, if the content of the pyrrole derivative is less than the lower limit, the cell survival rate may be excellent, but the effect of improving or treating gastrointestinal diseases may not be achieved to a desired extent. Conversely, if the content exceeds the upper limit, the effect of improving or treating gastrointestinal diseases may not increase as the concentration increases, or the composition may be toxic.

Hereinafter, the present invention will be described in detail by the following embodiments. However, the following embodiments are only for illustrating the present invention, and it will be apparent to those skilled in the art that the scope of the present invention is not limited to these embodiments.

### Working Examples, Preparation Examples and Comparative Examples

Analysis of the compounds prepared in the examples below was performed as follows. Nuclear magnetic resonance (NMR) spectrum analysis was performed on Varian 300MHz, JEOL 500MHz, and Bruker 700MHz spectrometers, chemical shift was analyzed in ppm, and column chromatography was performed on silica gel (Merck, 70-230 mesh). In addition, the starting materials for each of the working examples or preparation examples were known compounds, purchased from TCI, etc., and were commercially available compounds.

### Preparation Example 1 corresponding to Scheme 1

### 1-1. Carbaldehyde intermediate

The compound of formula 2 in Scheme 1 was suspended in tetrahydrofuran or chloroform. The reaction mixture was cooled to 0°C, and added with sodium hydride (1.5 eq) and sulfonyl chloride (1.5 eq), and stirred at 20±5°C for 30 minutes. Ethyl acetate and purified water were added to the reaction mixture to extract the organic layer. It was dried over sodium sulfate and filtered, and the filtrate was concentrated under reduced pressure. The concentrated residue was subjected to column chromatography in n-hexane:ethyl acetate = 3:1 to obtain the compound of formula 3 shown in Scheme 1.

### 1-2. Final compound

The compound of Preparation Example 1-1 was suspended in tetrahydrofuran and methanol. The reaction mixture was added with Deuterated methylamine hydrochloride (6 eq) and sodium hydrogen boride cyanide (3 eq) and stirred at 20 ± 5°C for 30 minutes. Purified water, aqueous sodium bicarbonate solution, and ethyl acetate were added to the reaction mixture to extract the organic layer. It was dried over sodium sulfate and filtered, and the filtrate was concentrated under reduced pressure. The concentrated residue was subjected to column chromatography in methylene chloride:methanol = 10:1 to obtain the compound of formula 4 shown in Scheme 1.

### Preparation Example 2 corresponding to Scheme 2

### 2-1. 5-aryl-1H-pyrrole-3-carbaldehyde

5-bromo-1H-pyrrole-3-carbaldehyde, aryl boronic acid (1.5 eq), and tetrakis(triphenylphosphine)palladium(0) (0.15 eq) were added to N,N-dimethylformamide and were stirred for 12 hours at 100±5°C in a microwave reactor. Purified water and ethyl acetate were added to the reaction mixture to extract the organic layer. The organic layer was dried over sodium sulfate and filtered, and the filtrate was concentrated under reduced pressure. The concentrated residue was subjected to column chromatography in n-hexane:ethyl acetate = 7:3 to obtain the compound of formula 6 shown in Scheme 2.

### 2-2. Carbaldehyde intermediate

The compound of Preparation Example 2-1 was suspended in tetrahydrofuran or chloroform. The reaction mixture was cooled to 0°C, and added with sodium hydride (1.5 eq) and sulfonyl chloride (1.5 eq), and stirred at 20±5°C for 30 minutes. Ethyl acetate and purified water were added to the reaction mixture to extract the organic layer. It was dried over sodium sulfate and filtered, and the filtrate was concentrated under reduced pressure. The concentrated residue was subjected to column chromatography in n-hexane:ethyl acetate = 3:1 to obtain the compound of formula 7 shown in Scheme 2.

### 2-3. Final compound

The compound of Preparation Example 2-2 was suspended in tetrahydrofuran and methanol. The reaction mixture was added with deuterated methylamine hydrochloride (6 eq) and sodium hydrogen boride cyanide (3 eq) and stirred at 20 ± 5°C for 30 minutes. Purified water, aqueous sodium bicarbonate solution, and ethyl acetate were added to the reaction mixture to extract the organic layer. It was dried over sodium sulfate and filtered, and the filtrate was concentrated under reduced pressure. The concentrated residue was subjected to column chromatography in methylene chloride:methanol = 10:1 to obtain the compound of formula 8 shown in Scheme 2.

### Preparation Example 3 corresponding to Scheme 3

### 3-1. Carboxylate compound

The compound of formula 9 in Scheme 3 was added to tetrahydrofuran. The reaction mixture was cooled to 0°C, added with sodium hydride (2.0 eq) and sulfonyl chloride (1.8 eq), and stirred at 70±5°C for 12 hours. A 1N aqueous hydrochloric acid solution and purified water were added to the reaction mixture to adjust the pH to 8 or higher, and methylene chloride was added to extract the organic layer. The organic layer was dried over sodium sulfate and filtered, and the filtrate was concentrated under reduced pressure. The concentrated residue was subjected to column chromatography in n-hexane:ethyl acetate = 4:1 to obtain the compound of formula 10 shown in Scheme 3.

### 3-2. Carbaldehyde compound

The carboxylate compound of Preparation Example 3-1 was dissolved in methylene chloride. The reaction mixture was cooled to 0°C, added with lithium aluminum hydride (3.0 eq), and stirred at 0±5°C for 10 minutes. The reaction was terminated with purified water and 1N sodium hydroxide solution, and the mixture was dried with sodium sulfate and filtered, and the filtrate was concentrated under reduced pressure. The concentrated residue was dissolved in methylene chloride. The reaction mixture was cooled to 0°C, added with Dess-Martin periodinane (2.0 eq), and stirred at 20±5°C for 30 minutes. The reaction was terminated, followed by addition of aqueous sodium bicarbonate solution and purified water to adjust the pH to 8 or higher. The mixture was added with methylene chloride to extract the organic layer, dried with sodium sulfate, and filtered, and the filtrate was concentrated under reduced pressure. The concentrated residue was subjected to column chromatography in methylene chloride:methanol = 8:1 to obtain formula 12 shown in Scheme 3.

### 3-3. Final compound

The compound of Preparation Example 3-2 was suspended in tetrahydrofuran and methanol. The reaction mixture was added with deuterated methylamine hydrochloride (6 eq) and sodium hydrogen boride cyanide (3 eq) and stirred at 20 ± 5°C for 30 minutes. Purified water, aqueous sodium bicarbonate solution, and ethyl acetate were added to the reaction mixture to extract the organic layer. The organic layer was dried over sodium sulfate and filtered, and the filtrate was concentrated under reduced pressure. The concentrated residue was subjected to column chromatography in methylene chloride:methanol = 10:1 to obtain the compound of formula 13 shown in Scheme 3.

### Example 1. N-((5-2-fluorophenyl)-1-((4-(trifluoromethyl)phenyl)sulfonyl)-1H-pyrrol-3-yl)methyl)methan-d₃-amine Preparation (corresponding to Scheme 1)

### 1-1. Preparation of 5-(2-fluorophenyl)-1-((4-(trifluoromethyl)phenyl)sulfonyl)-1H-pyrrole-3-carbaldehyde compound

The compound of Example 1-1, as the title compound, was obtained according to Preparation Example 1-1. (Yield: 41%)

¹H NMR (500 MHz, CDCl₃) δ 9.90 (s, 1H), 8.12 (d, J = 1.9 Hz, 1H), 7.48 - 7.39 (m, 2H), 7.33 - 7.29 (m, 1H), 7.25 (dd, J = 1.7, 1.0 Hz, 1H), 7.18 - 7.14 (m, 2H), 7.06 - 7.00 (m, 2H), 6.67 (d, J = 1.8 Hz, 1H).

### 1-2. Preparation of N-((5-(2-fluorophenyl)-1-((4-(trifluoromethyl)phenyl)sulfonyl)-1H-pyrrol-3-yl)methyl)methan-d₃-amine compound

The compound of Example 1-1 was prepared into the compound of Example 1, as the title compound, according to Preparation Example 1-2. (Yield: 39%)

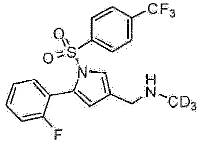

¹H NMR (500 MHz, CDCl₃) δ 8.15 (d, J = 1.8 Hz, 1H), 7.67 (d, J = 8.3 Hz, 2H), 7.60 (d, J = 8.2 Hz, 2H), 7.46 - 7.40 (m, 1H), 7.15 - 7.10 (m, 2H), 7.01 - 6.96 (m, 1H), 6.67 (d, J = 1.8 Hz, 1H), 3.71 (s, 2H).

### Example 2. Preparation of N-((5-(2-fluorophenyl)-1-tosyl-1H-pyrrol-3-yl)methyl)methan-d₃-amine (corresponding to Scheme 1)

### 2-1. Preparation of 5-(2-fluorophenyl)-1-tosyl-1H-pyrrole-3-carbaldehyde compound

The compound of Example 2-1, as the title compound, was obtained according to Preparation Example 1-1. (Yield: 67%)

¹H NMR (500 MHz, DMSO-d₆) δ 9.85 (s, 1H), 8.52 (d, J = 1.8 Hz, 1H), 7.58 - 7.50 (m, 1H), 7.43 - 7.36 (m, 4H), 7.25 - 7.18 (m, 2H), 7.09 (td, J = 7.5, 1.7 Hz, 1H), 6.65 (d, J = 1.8 Hz, 1H), 2.38 (s, 3H).

### 2-2. Preparation of N-((5-(2-fluorophenyl)-1-tosyl-1H-pyrrol-3-yl)methyl)methan-d₃-amine compound

The compound of Example 2-1 was prepared into the compound of Example 2, as the title compound, according to Preparation Example 1-2. (Yield: 55%)

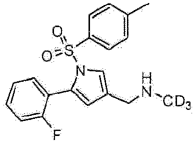

¹H NMR (500 MHz, CDCl₃) δ 7.39 - 7.34 (m, 2H), 7.32 (s, 1H), 7.30 (s, 1H), 7.18 - 7.13 (m, 3H), 7.10 (t, J = 7.4 Hz, 1H), 7.02 (t, J = 8.9 Hz, 1H), 6.23 (d, J = 1.6 Hz, 1H), 3.63 (s, 2H), 2.37 (s, 3H).

### Example 3. Preparation of N-((5-(2-fluorophenyl)-1-((4-methoxyphenyl)sulfonyl)-1H-pyrrol-3-yl)methyl)methan-d₃-amine (corresponding to Scheme 1)

### 3-1. Preparation of 5-(2-fluorophenyl)-1-((4-methoxyphenyl)sulfonyl)-1H-pyrrole-3-carbaldehyde compound

The compound of Example 3-1, as the title compound, was obtained according to Preparation Example 1-1. (Yield: 70%)

¹H NMR (500 MHz, DMSO-d₆) δ 9.85 (s, 1H), 8.50 (d, J = 1.8 Hz, 1H), 7.57 - 7.50 (m, 1H), 7.46 - 7.40 (m, 2H), 7.25 - 7.20 (m, 2H), 7.11 (td, J = 7.3, 1.5 Hz, 1H), 7.09 - 7.05 (m, 2H), 6.64 (d, J = 1.8 Hz, 1H), 3.84 (s, 3H).

### 3-2. Preparation of N-((5-(2-fluorophenyl)-1-((4-methoxyphenyl)sulfonyl)-1H-pyrrol-3-yl)methyl)methan-d₃-amine compound

The compound of Example 3-1 was prepared into the compound of Example 3, as the title compound, according to Preparation Example 1-2. (Yield: 57%)

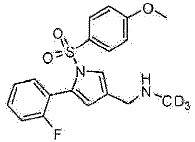

¹H NMR (500 MHz, CDCl₃) δ 7.39 - 7.33 (m, 4H), 7.20 - 7.15 (m, 1H), 7.10 (t, J = 7.5 Hz, 1H), 7.02 (t, J = 8.9 Hz, 1H), 6.80 (d, J = 8.9 Hz, 2H), 6.24 (d, J = 1.3 Hz, 1H), 3.82 (s, 3H), 3.65 (s, 2H).

### Example 4. Preparation of 4-((2-(2-fluorophenyl)-4-(((methyl-d3)amino)methyl)-1H-pyrrol-1-yl)sulfonyl)benzonitrile (corresponding to Scheme 1)

### 4-1. Preparation of 4-((2-(2-fluorophenyl)-4-formyl-1H-pyrrol-1-yl)sulfonyl)benzonitrile compound

The compound of Example 4-1, as the title compound, was obtained according to Preparation Example 1-1. (Yield: 64%)

¹H NMR (500 MHz, DMSO-d₆) δ 9.87 (s, 1H), 8.60 (d, J = 1.7 Hz, 1H), 8.08 (d, J = 8.6 Hz, 2H), 7.70 (d, J = 8.6 Hz, 2H), 7.58 - 7.52 (m, 1H), 7.22 (q, J = 8.0 Hz, 2H), 7.11 (td, J = 7.5, 1.6 Hz, 1H), 6.72 (d, J = 1.7 Hz, 1H).

### 4-2. Preparation of 4-((2-(2-fluorophenyl)-4-(((methyl-d3)amino)methyl)-1H-pyrrol-1-yl)sulfonyl)benzonitrile compound

The compound of Example 4-1 was prepared into the compound of Example 4, as the title compound, according to Preparation Example 1-2. (Yield: 64%)

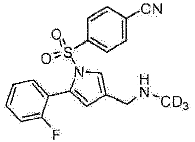

¹H NMR (300 MHz, CDCl₃) δ 7.68 - 7.64 (m, 2H), 7.54 - 7.50 (m, 2H), 7.43 - 7.35 (m, 2H), 7.20 - 7.10 (m, 2H), 7.03 (t, J = 9.0 Hz, 1H), 6.26 (d, J = 1.8 Hz, 1H), 3.62 (s, 2H).

### Example 5. Preparation of N-((1-((4-bromophenyl)sulfonyl)-5-(2-fluorophenyl)-1H-pyrrol-3-yl)methyl)methan-d₃-amine (corresponding to Scheme 1)

### 5-1. Preparation of 1-((4-bromophenyl)sulfonyl)-5-(2-fluorophenyl)-1H-pyrrole-3-carbaldehyde compound

The compound of Example 5-1, as the title compound, was obtained according to Preparation Example 1-1. (Yield: 65%)

¹H NMR (500 MHz, DMSO-d₆) δ 9.86 (s, 1H), 8.56 (s, 1H), 7.81 (d, J = 8.4 Hz, 2H), 7.55 (q, J = 6.7 Hz, 1H), 7.44 (d, J = 8.5 Hz, 2H), 7.26 - 7.19 (m, 2H), 7.13 (t, J = 7.2 Hz, 1H), 6.69 (s, 1H).

### 5-2. Preparation of N-((1-((4-bromophenyl)sulfonyl)-5-(2-fluorophenyl)-1H-pyrrol-3-yl)methyl)methan-d₃-amine compound

The compound of Example 5-1 was prepared into the compound of Example 5, as the title compound, according to Preparation Example 1-2. (Yield: 37%)

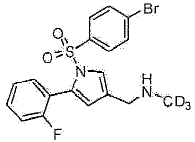

¹H NMR (500 MHz, CD₃OD) δ 7.74 (d, J = 1.5 Hz, 1H), 7.67 (d, J = 8.6 Hz, 2H), 7.53 - 7.46 (m, 1H), 7.35 (d, J = 8.6 Hz, 2H), 7.19 (t, J = 7.5 Hz, 1H), 7.14 - 7.06 (m, 2H), 6.41 (d, J = 1.8 Hz, 1H), 4.08 (s, 2H).

### Example 6. Preparation of N-((1-((4-chlorophenyl)sulfonyl)-5-(2-fluorophenyl)-1H-pyrrol-3-yl)methyl)methan-d₃-amine (corresponding to Scheme 1)

### 6-1. Preparation of 1-((4-chlorophenyl)sulfonyl)-5-(2-fluorophenyl)-1H-pyrrole-3-carbaldehyde compound

The compound of Example 6-1, as the title compound, was obtained according to Preparation Example 1-1. (Yield: 69%)

¹H NMR (500 MHz, CDCl₃) δ 9.88 (s, 1H), 8.10 (d, J = 1.8 Hz, 1H), 7.44 (ddd, J = 13.2, 6.7, 3.8 Hz, 1H), 7.38 - 7.31 (m, 4H), 7.17 - 7.13 (m, 2H), 7.01 (t, J = 8.8 Hz, 1H), 6.65 (d, J = 1.8 Hz, 1H).

### 6-2. Preparation of N-((1-((4-chlorophenyl)sulfonyl)-5-(2-fluorophenyl)-1H-pyrrol-3-yl)methyl)methan-d₃-amine compound

The compound of Example 6-1 was prepared into the compound of Example 6, as the title compound, according to Preparation Example 1-2. (Yield: 64%)

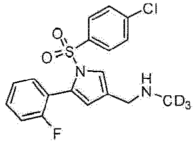

¹H NMR (300 MHz, CDCl₃) δ 7.54 - 7.28 (m, 6H), 7.18 - 6.99 (m, 3H), 6.26 (d, J = 1.8 Hz, 1H), 3.63 (s, 2H).

### Example 7. Preparation of N-((5-(2-fluorophenyl)-1-((4-fluorophenyl)sulfonyl)-1H-pyrrol-3-yl)methyl)methan-d₃-amine (corresponding to Scheme 1)

### 7-1. Preparation of 5-(2-fluorophenyl)-1-((4-fluorophenyl)sulfonyl)-1H-pyrrole-3-carbaldehyde compound

The compound of Example 7-1, as the title compound, was obtained according to Preparation Example 1-1. (Yield: 54%)

¹H NMR (500 MHz, DMSO-d₆) δ 9.86 (s, 1H), 8.56 (d, J = 1.3 Hz, 1H), 7.59 (dd, J = 8.7, 4.9 Hz, 2H), 7.56 - 7.51 (m, 1H), 7.44 (t, J = 8.7 Hz, 2H), 7.25 - 7.18 (m, 2H), 7.11 (t, J = 6.9 Hz, 1H), 6.69 (s, 1H).

### 7-2. Preparation of N-((5-(2-fluorophenyl)-1-((4-fluorophenyl)sulfonyl)-1H-pyrrol-3-yl)methyl)methan-d₃-amine compound

The compound of Example 7-1 was prepared into the compound of Example 7, as the title compound, according to Preparation Example 1-2. (Yield: 50%)

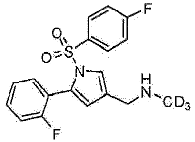

¹H NMR (300 MHz, CDCl₃) δ 7.41 - 7.29 (m, 4H), 7.16 - 7.05 (m, 2H), 7.05 - 6.99 (m, 3H), 6.23 (d, J = 1.8 Hz, 1H), 3.61 (s, 2H).

### Example 8. Preparation of N-((5-(2-fluorophenyl)-1-((4-(trifluoromethoxy)phenyl)sulfonyl)-1H-pyrrol-3-yl)methyl)methan-d₃-amine (corresponding to Scheme 1)

### 8-1. Preparation of 5-(2-fluorophenyl)-1-((4-(trifluoromethoxy)phenyl)sulfonyl)-1H-pyrrole-3-carbaldehyde compound

The compound of Example 8-1, as the title compound, was obtained according to Preparation Example 1-1. (Yield: 43%)

¹H NMR (500 MHz, CDCl₃) δ 9.90 (s, 1H), 8.13 (d, J = 1.8 Hz, 1H), 7.47 - 7.41 (m, 3H), 7.21 - 7.13 (m, 4H), 6.98 (t, J = 8.9 Hz, 1H), 6.67 (d, J = 1.8 Hz, 1H).

### 8-2. Preparation of N-((5-(2-fluorophenyl)-1-((4-(trifluoromethoxy)phenyl)sulfonyl)-1H-pyrrol-3-yl)methyl)methan-d₃-amine compound

The compound of Example 8-1 was prepared into the compound of Example 8, as the title compound, according to Preparation Example 1-2. (Yield: 36%)

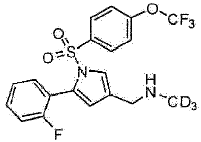

¹H NMR (300 MHz, CD₃OD) δ 7.56 (ddd, J = 9.7, 4.7, 2.3 Hz, 2H), 7.52 - 7.40 (m, 2H), 7.36 (d, J = 8.1 Hz, 2H), 7.19 - 7.01 (m, 3H), 6.34 (d, J = 1.9 Hz, 1H), 3.78 (s, 2H).

### Example 9. Preparation of N-((1-((4'-fluoro-[1,1'-biphenyl]-4-yl)sulfonyl)-5-(2-fluorophenyl)-1H-pyrrol-3-yl)methyl)methan-d₃-amine (corresponding to Scheme 1)

### 9-1. Preparation of 1-((4'-fluoro-[1,1'-biphenyl]-4-yl)sulfonyl)-5-(2-fluorophenyl)-1H-pyrrole-3-carbaldehyde compound

The compound of Example 9-1, as the title compound, was obtained according to Preparation Example 1-1. (Yield: 62%)

¹H NMR (300 MHz, DMSO-d₆) δ 9.87 (s, 1H), 8.59 (d, J = 1.9 Hz, 1H), 7.89 - 7.83 (m, 2H), 7.83 - 7.76 (m, 2H), 7.60 - 7.51 (m, 3H), 7.40 - 7.31 (m, 2H), 7.27 - 7.18 (m, 2H), 7.17 - 7.09 (m, 1H), 6.69 (d, J = 1.8 Hz, 1H).

### 9-2. Preparation of N-((1-((4'-fluoro-[1,1'-biphenyll-4-yl)sulfonyl)-5-(2-fluorophenyl)-1H-pyrrol-3-yl)methyl) methane-d₃-amine compound

The compound of Example 9-1 was prepared into the compound of Example 9, as the title compound, according to Preparation Example 1-2. (Yield: 34%)

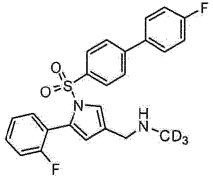

¹H NMR (300 MHz, CD₃OD) δ 7.72 - 7.60 (m, 4H), 7.56 - 7.37 (m, 4H), 7.26 - 7.01 (m, 5H), 6.30 (d, J = 1.7 Hz, 1H), 3.65 (s, 2H).

### Example 10. Preparation of N-((1-((4-chlorophenyl)sulfonyl)-5-(3-fluorophenyl)-1H-pyrrol-3-yl)methyl)methan-d₃-amine (corresponding to Scheme 2)

### 10-1. Preparation of 1-((4-chlorophenyl)sulfonyl)-5-(3-fluorophenyl)-1H-pyrrole-3-carbaldehyde compound

The compound of Example 10-1, as the title compound, was obtained according to Preparation Example 2-2. (Yield: 33%)

¹H NMR (500 MHz, CDCl₃) δ 9.88 (s, 1H) 8.09 (d, J = 1.9 Hz, 1H), 7.37 - 7.27 (m, 5H), 7.13 (tdd, J = 8.4, 2.6, 0.9 Hz, 1H), 7.01 - 6.94 (m, 1H), 6.90 (ddd, J = 9.4, 2.5, 1.6 Hz, 1H), 6.61 (d, J = 1.9 Hz, 1H).

### 10-2. Preparation of N-((1-((4-chlorophenyl)sulfonyl)-5-(3-fluorophenyl)-1H-pyrrol-3-yl)methyl)methan-d₃-amine compound

The compound of Example 10-1 was prepared into the compound of Example 10, as the title compound, according to Preparation Example 2-3. (Yield: 35%)

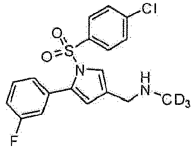

¹H NMR (500 MHz, DMSO-d₆) δ 7.60 (dd, J = 8.6, 6.9 Hz, 2H), 7.44 - 7.38 (m, 4H), 7.28 - 7.20 (m, 1H), 7.07 - 7.00 (m, 2H), 6.37 (d, J = 1.5 Hz, 1H), 3.49 (s, 2H).

### Example 11. Preparation of N-((5-(3-fluorophenyl)-1-((4-methoxyphenyl)sulfonyl)-1H-pyrrol-3-yl)methyl)methan-d₃-amine (corresponding to Scheme 2)

### 11-1. Preparation of 5-(3-fluorophenyl)-1-((4-methoxyphenyl)sulfonyl)-1H-pyrrole-3-carbaldehyde compound

The compound of Example 11-1, as the title compound, was obtained according to Preparation Example 2-2. (Yield: 42%)

¹H NMR (500 MHz, DMSO-d₆) δ 9.86 (s, 1H), 8.47 (d, J = 1.8 Hz, 1H), 7.44 - 7.37 (m, 3H), 7.32 - 7.26 (m, 1H), 7.06 - 7.02 (m, 2H), 7.01 - 6.95 (m, 2H), 6.62 (d, J = 1.8 Hz, 1H), 3.83 (s, 3H).

### 11-2. Preparation of N-((5-(3-fluorophenyl)-1-((4-methoxyphenyl)sulfonyl)-1H-pyrrol-3-yl)methyl)methan-d₃-amine compound

The compound of Example 11-1 was prepared into the compound of Example 11, as the title compound, according to Preparation Example 2-3. (Yield: 30%)

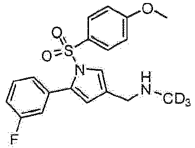

¹H NMR (500 MHz, DMSO-d₆) δ 7.45 (d, J = 1.0 Hz, 1H), 7.40 (td, J = 8.0, 6.3 Hz, 1H), 7.36 - 7.32 (m, 2H), 7.24 (td, J = 8.8, 2.6 Hz, 1H), 7.05 - 6.98 (m, 4H), 6.36 (d, J = 1.8 Hz, 1H), 3.81 (s, 3H), 3.59 (s, 2H).

### Example 12. Preparation of N-((5-(2,4-difluorophenyl)-1-((4-fluorophenyl)sulfonyl)-1H-pyrrol-3-yl)methyl)methan-d₃-amine (corresponding to Scheme 2)

### 12-1. Preparation of 5-(2,4-difluorophenyl)-1-((4-fluorophenyl)sulfonyl)-1H-pyrrole-3-carbaldehyde compound

The compound of Example 12-1, as the title compound, was obtained according to Preparation Example 2-2. (Yield: 94%)

¹H NMR (500 MHz, CDCl₃) δ 9.89 (s, 1H), 8.12 (d, J = 1.9 Hz, 1H), 7.51 - 7.41 (m, 2H), 7.16 (td, J = 8.3, 6.4 Hz, 1H), 7.13 - 7.08 (m, 2H), 6.93 - 6.87 (m, 1H), 6.78 (td, J = 9.0, 2.5 Hz, 1H), 6.65 (d, J = 1.9 Hz, 1H).

### 12-2. Preparation of N-((5-(2,4-difluorophenyl)-1-((4-fluorophenyl)sulfonyl)-1H-pyrrol-3-yl)methyl)methan-d₃-amine compound

The compound of Example 12-1 was prepared into the compound of Example 12, as the title compound, according to Preparation Example 2-3. (Yield: 35%)

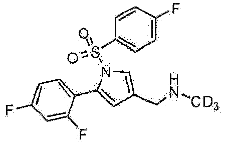

¹H NMR (500 MHz, CD₃OD) δ 7.53 - 7.47 (m, 2H), 7.23 - 7.16 (m, 2H), 7.13 - 7.07 (m, 1H), 6.96 - 6.90 (m, 2H), 6.29 (d, J = 1.9 Hz, 1H), 3.62 (s, 2H).

### Example 13. Preparation of N-((1-((4-bromophenyl)sulfonyl)-5-(2,4-difluorophenyl)-1H-pyrrol-3-yl)methyl)methan-d₃-amine (corresponding to Scheme 2)

### 13-1. Preparation of 1-((4-bromophenyl)sulfonyl)-5-(2,4-difluorophenyl)-1H-pyrrole-3-carbaldehyde compound

The compound of Example 13-1, as the title compound, was obtained according to Preparation Example 2-2. (Yield: 83%)

¹H NMR (500 MHz, CDCl₃) δ 9.89 (s, 1H), 8.10 (d, J = 1.8 Hz, 1H), 7.62 - 7.54 (m, 2H), 7.32 - 7.27 (m, 2H), 7.14 (td, J = 8.3, 6.3 Hz, 1H), 6.94 - 6.87 (m, 1H), 6.79 (td, J = 9.0, 2.5 Hz, 1H), 6.66 (d, J = 1.8 Hz, 1H).

### 13-2. Preparation of N-((1-((4-bromophenyl)sulfonyl)-5-(2,4-difluorophenyl)-1H-pyrrol-3-yl)methyl)methan-d₃-amine compound

The compound of Example 13-1 was prepared into the compound of Example 13, as the title compound, according to Preparation Example 2-3. (Yield: 30%)

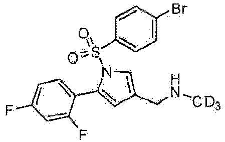

¹H NMR (500 MHz, CD₃OD) δ 7.73 - 7.61 (m, 2H), 7.54 - 7.42 (m, 1H), 7.40 - 7.30 (m, 2H), 7.14 (ddd, J = 10.2, 7.2, 4.3 Hz, 1H), 6.96 (ddt, J = 9.8, 4.5, 2.6 Hz, 2H), 6.32 (d, J = 1.9 Hz, 1H), 3.67 - 3.58 (m, 2H).

### Example 14. Preparation of N-((5-(2,4-difluorophenyl)-1-((4-(trifluoromethyl)phenyl)sulfonyl)-1H-pyrrol-3-yl)methyl)methan-d₃-amine (corresponding to Scheme 2)

### 14-1. Preparation of 5-(2,4-difluorophenyl)-1-((4-(trifluoromethyl)phenyl)sulfonyl)-1H-pyrrole-3-carbaldehyde compound

The compound of Example 14-1, as the title compound, was obtained according to Preparation Example 2-2. (Yield: 18%)

¹H NMR (500 MHz, CDCl₃) δ 9.89 (s, 1H), 8.12 (d, J = 1.8 Hz, 1H), 7.70 (d, J = 8.4 Hz, 2H), 7.58 (d, J = 8.3 Hz, 2H), 7.14 (td, J = 8.3, 6.4 Hz, 1H), 6.90 (td, J = 8.0, 2.4 Hz, 1H), 6.79 - 6.71 (m, 1H), 6.66 (d, J = 1.8 Hz, 1H).

### 14-2. Preparation of N-((5-(2,4-difluorophenyl)-1-((4-(trifluoromethyl)phenyl)sulfonyl)-1H-pyrrol-3-yl)methyl)methan-d₃-amine compound

The compound of Example 14-1 was prepared into the compound of Example 14, as the title compound, according to Preparation Example 2-3. (Yield: 43%)

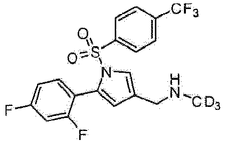

¹H NMR (500 MHz, CD₃OD) δ 7.84 (d, J = 8.3 Hz, 2H), 7.70 (d, J = 8.3 Hz, 2H), 7.61 - 7.48 (m, 1H), 7.22 - 7.11 (m, 1H), 7.04 - 6.89 (m, 2H), 6.36 (d, J = 1.9 Hz, 1H), 3.67 (s, 2H).

### Example 15. Preparation of N-((5-(2,4-difluorophenyl)-1-((4-(trifluoromethoxy)phenyl)sulfonyl)-1H-pyrrol-3-yl)methyl)methan-d₃-amine (corresponding to Scheme 2)

### 15-1. Preparation of 5-(2,4-difluorophenyl)-1-((4-(trifluoromethoxy)phenyl)sulfonyl)-1H-pyrrole-3-carbaldehyde compound

The compound of Example 15-1, as the title compound, was obtained according to Preparation Example 2-2. (Yield: 81%)

¹H NMR (500 MHz, CDCl₃) δ 9.90 (s, 1H), 8.13 (d, J = 1.8 Hz, 1H), 7.52 - 7.46 (m, 2H), 7.24 (dd, J = 8.9, 0.8 Hz, 2H), 7.16 (td, J = 8.3, 6.4 Hz, 1H), 6.94 - 6.86 (m, 1H), 6.75 (td, J = 9.0, 2.5 Hz, 1H), 6.66 (d, J = 1.8 Hz, 1H).

### 15-2. Preparation of N-((5-(2,4-difluorophenyl)-1-((4-(trifluoromethoxy)phenyl)sulfonyl)-1H-pyrrol-3-yl)methyl)methan-d₃-amine compound

The compound of Example 15-1 was prepared into the compound of Example 15, as the title compound, according to Preparation Example 2-3. (Yield: 45%)

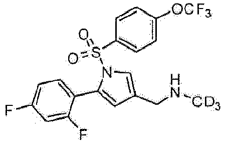

¹H NMR (500 MHz, CD₃OD) δ 7.63 - 7.55 (m, 2H), 7.51 - 7.46 (m, 1H), 7.39 (dd, J = 8.9, 0.9 Hz, 2H), 7.15 (ddd, J = 12.2, 7.1, 4.4 Hz, 1H), 7.00 - 6.90 (m, 2H), 6.32 (d, J = 1.9 Hz, 1H), 3.60 (s, 2H).

### Example 16. Preparation of N-((5-(2,4-difluorophenyl)-1-tosyl-1H-pyrrol-3-yl)methyl)methane-d₃-amine (corresponding to Scheme 2)

### 16-1. Preparation of 5-(2,4-difluorophenyl)-1-tosyl-1H-pyrrole-3-carbaldehyde compound

The compound of Example 16-1, as the title compound, was obtained according to Preparation Example 2-2. (Yield: 46%)

¹H NMR (500 MHz, CDCl₃) δ 9.88 (s, 1H), 8.11 (d, J = 1.8 Hz, 1H), 7.34 - 7.30 (m, 2H), 7.23 - 7.19 (m, 2H), 7.12 (td, J = 8.3, 6.4 Hz, 1H), 6.90 - 6.85 (m, 1H), 6.77 (td, J = 9.0, 2.5 Hz, 1H), 6.63 (d, J = 1.8 Hz, 1H), 2.42 (s, 3H).

### 16-2. Preparation of N-((5-(2,4-difluorophenyl)-1-tosyl-1H-pyrrol-3-yl)methyl)methane-d₃-amine compound

The compound of Example 16-1 was prepared into the compound of Example 16, as the title compound, according to Preparation Example 2-3. (Yield: 37%)

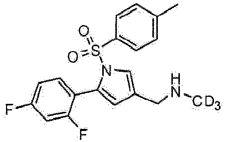

¹H NMR (500 MHz, CD₃OD) δ 7.47 - 7.44 (m, 1H), 7.36 - 7.31 (m, 2H), 7.30 - 7.25 (m, 2H), 7.09 (ddd, J = 14.6, 7.3, 4.1 Hz, 1H), 6.93 (dq, J = 2.6, 1.9 Hz, 2H), 6.27 (d, J = 1.9 Hz, 1H), 3.59 (d, J = 3.6 Hz, 2H), 2.40 (d, J = 6.2 Hz, 3H).

### Example 17. Preparation of N-((5-(2,4-difluorophenyl)-1-((4-methoxyphenyl)sulfonyl)-1H-pyrrol-3-yl)methyl)methan-d₃-amine (corresponding to Scheme 2)

### 17-1. Preparation of 5-(2,4-difluorophenyl)-1-((4-methoxyphenyl)sulfonyl)-1H-pyrrole-3-carbaldehyde compound

The compound of Example 17-1, as the title compound, was obtained according to Preparation Example 2-2. (Yield: 76%)

¹H NMR (500 MHz, CDCl₃) δ 9.93 - 9.83 (m, 1H), 8.11 (d, J = 1.9 Hz, 1H), 7.38 - 7.33 (m, 2H), 7.15 (td, J = 8.3, 6.4 Hz, 1H), 6.92 - 6.83 (m, 3H), 6.77 (td, J = 9.0, 2.5 Hz, 1H), 6.62 (d, J = 1.8 Hz, 1H), 3.86 (s, 3H).

### 17-2. Preparation of N-((5-(2,4-difluorophenyl)-1-((4-methoxyphenyl)sulfonyl)-1H-pyrrol-3-yl)methyl)methan-d₃-amine compound

The compound of Example 17-1 was prepared into the compound of Example 17, as the title compound, according to Preparation Example 2-3. (Yield: 36%)

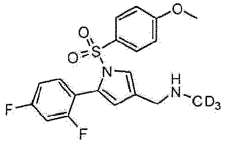

¹H NMR (500 MHz, CD₃OD) δ 7.48 - 7.45 (m, 1H), 7.41 - 7.37 (m, 2H), 7.14 - 7.08 (m, 1H), 6.98 - 6.91 (m, 4H), 6.27 (d, J = 1.9 Hz, 1H), 3.85 (s, 3H), 3.62 (s, 2H).

### Example 18. Preparation of 4-((2-(2,4-difluorophenyl-4-(((methyl-d₃)amino)methyl)-1H-pyrrol-1-yl)sulfonyl)benzonitrile (corresponding to Scheme 2)

### 18-1. Preparation of 4-((2-(2,4-difluorophenyl)-4-formyl-1H-pyrrol-1-yl)sulfonyl)benzonitrile compound

The compound of Example 18-1, as the title compound, was obtained according to Preparation Example 2-2. (Yield: 73%)

¹H NMR (500 MHz, CDCl₃) δ 9.89 (s, 1H), 8.11 (d, J = 1.8 Hz, 1H), 7.77 - 7.68 (m, 2H), 7.62 - 7.53 (m, 2H), 7.16 (td, J = 8.3, 6.3 Hz, 1H), 6.95 - 6.88 (m, 1H), 6.78 (td, J = 9.0, 2.5 Hz, 1H), 6.68 (d, J = 1.8 Hz, 1H).

### 18-2. Preparation of 4-((2-(2,4-difluorophenyl-4-(((methyl-d3)amino)methyl)-1H-pyrrol-1-yl)sulfonyl)benzonitrile compound

The compound of Example 18-1 was prepared into the compound of Example 18, as the title compound, according to Preparation Example 2-3. (Yield: 45%)

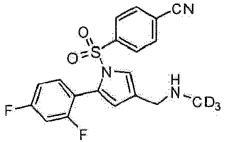

¹H NMR (500 MHz, CD₃OD) δ 7.91 - 7.83 (m, 2H), 7.66 - 7.60 (m, 2H), 7.54 - 7.49 (m, 1H), 7.13 (ddd, J = 8.0, 7.3, 3.7 Hz, 1H), 7.01 - 6.94 (m, 2H), 6.35 (d, J = 1.8 Hz, 1H), 3.64 - 3.61 (m, 2H).

### Example 19. Preparation of N-((5-(3-fluorophenyl)-1-((4-fluorophenyl)sulfonyl)-1H-pyrrol-3-yl)methyl)methan-d₃-amine (corresponding to Scheme 2)

### 19-1. Preparation of 5-(3-fluorophenyl)-1-((4-fluorophenyl)sulfonyl)-1H-pyrrole-3-carbaldehyde compound

The compound of Example 19-1, as the title compound, was obtained according to Preparation Example 2-2. (Yield: 71%)

¹H NMR (500 MHz, DMSO-d₆) δ 9.88 (s, 1H), 8.53 (d, J = 1.8 Hz, 1H), 7.61 - 7.54 (m, 2H), 7.44 - 7.36 (m, 3H), 7.32 - 7.27 (m, 1H), 6.99 (dd, J = 9.2, 2.0 Hz, 2H), 6.66 (d, J = 1.8 Hz, 1H).

### 19-2. Preparation of N-((5-(3-fluorophenyl)-1-((4-fluorophenyl)sulfonyl)-1H-pyrrol-3-yl)methyl)methan-d₃-amine compound

The compound of Example 19-1 was prepared into the compound of Example 19, as the title compound, according to Preparation Example 2-3. (Yield: 27%)

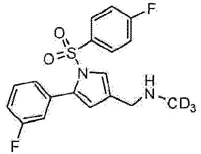

¹H NMR (500 MHz, CDCl₃) δ 7.38 (ddd, J = 11.6, 6.7, 4.3 Hz, 3H), 7.27 (dd, J = 8.0, 2.0 Hz, 1H), 7.03 (ddd, J = 26.0, 12.8, 5.3 Hz, 4H), 6.96 - 6.90 (m, 1H), 6.21 (s, 1H), 3.62 (s, 2H), 2.39 (s, 1H).

### Example 20. Preparation of N-((1-((4-bromophenyl)sulfonyl)-5-(3-fluorophenyl)-1H-pyrrol-3-yl)methyl)methan-d₃-amine (corresponding to Scheme 2)

### 20-1. Preparation of 1-((4-bromophenyl)sulfonyl)-5-(3-fluorophenyl)-1H-pyrrole-3-carbaldehyde compound

The compound of Example 20-1, as the title compound, was obtained according to Preparation Example 2-2. (Yield: 34%)

¹H NMR (500 MHz, DMSO-d₆) δ 9.87 (s, 1H), 8.52 (d, J = 1.7 Hz, 1H), 7.82 - 7.75 (m, 2H), 7.45 - 7.38 (m, 3H), 7.30 (ddd, J = 8.6, 2.9, 1.5 Hz, 1H), 7.05 - 6.96 (m, 2H), 6.67 (d, J = 1.7 Hz, 1H).

### 20-2. Preparation of N-((1-((4-bromophenyl)sulfonyl)-5-(3-fluorophenyl)-1H-pyrrol-3-yl)methyl)methan-d₃-amine compound

The compound of Example 20-1 was prepared into the compound of Example 20, as the title compound, according to Preparation Example 2-3. (Yield: 29%)

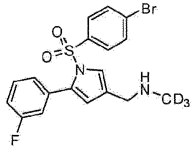

¹H NMR (500 MHz, CD₃OD) δ 7.60 - 7.57 (m, 2H), 7.47 - 7.45 (m, 1H), 7.34 - 7.29 (m, 1H), 7.29 - 7.25 (m, 2H), 7.11 (tdd, J = 8.7, 2.6, 0.9 Hz, 1H), 6.99 (ddd, J = 7.7, 1.4, 1.0 Hz, 1H), 6.95 (ddd, J = 9.9, 2.5, 1.6 Hz, 1H), 6.29 (d, J = 1.9 Hz, 1H), 3.63 (s, 2H).

### Example 21. Preparation of N-((5-(3-fluorophenyl)-1-tosyl-1H-pyrrol-3-yl)methyl)methan-d₃-amine (corresponding to Scheme 2)

### 21-1. Preparation of 5-(3-fluorophenyl)-1-tosyl-1H-pyrrole-3-carbaldehyde compound

The compound of Example 21-1, as the title compound, was obtained according to Preparation Example 2-2. (Yield: 71%)

¹H NMR (500 MHz, DMSO-d₆) δ 9.86 (s, 1H), 8.49 (d, J = 1.8 Hz, 1H), 7.43 - 7.34 (m, 5H), 7.31 - 7.26 (m, 1H), 7.01 - 6.98 (m, 1H), 6.98 - 6.94 (m, 1H), 6.63 (d, J = 1.8 Hz, 1H), 2.36 (s, 3H).

### 21-2. Preparation of N-((5-(3-fluorophenyl)-1-tosyl-1H-pyrrol-3-yl)methyl)methan-d₃-amine compound

The compound of Example 21-1 was prepared into the compound of Example 21, as the title compound, according to Preparation Example 2-3. (Yield: 10%)

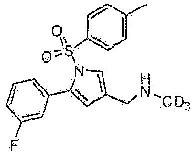

¹H NMR (500 MHz, CD₃OD) δ 7.45 - 7.42 (m, 1H), 7.32 - 7.26 (m, 1H), 7.26 - 7.23 (m, 2H), 7.19 (d, J = 8.1 Hz, 2H), 7.08 (tdd, J = 8.7, 2.6, 0.9 Hz, 1H), 7.00 - 6.96 (m, 1H), 6.90 (ddd, J = 10.0, 2.5, 1.6 Hz, 1H), 6.24 (d, J = 1.9 Hz, 1H), 3.59 (s, 2H), 2.34 (s, 3H).

### Example 22. Preparation of 4-((2-(3-fluorophenyl)-4-(((methyl-d3)amino)methyl)-1H-pyrrol-1-yl)sulfonyl)benzonitrile (corresponding to Scheme 2)

### 22-1. Preparation of 4-((2-(3-fluorophenyl)-4-formyl-1H-pyrrol-1-yl)sulfonyl)benzonitrile compound

The compound of Example 22-1, as the title compound, was obtained according to Preparation Example 2-2. (Yield: 89%)

¹H NMR (500 MHz, CDCl₃) δ 9.90 (s, 1H), 8.11 (t, J = 2.2 Hz, 1H), 7.71 - 7.64 (m, 2H), 7.52 - 7.46 (m, 2H), 7.31 (tt, J = 10.6, 5.3 Hz, 1H), 7.16 (tdd, J = 8.4, 2.6, 1.0 Hz, 1H), 6.95 (ddd, J = 7.6, 1.5, 1.1 Hz, 1H), 6.91 (ddd, J = 9.3, 2.5, 1.6 Hz, 1H), 6.64 (d, J = 1.9 Hz, 1H).

### 22-2. Preparation of 4-((2-(3-fluorophenyl)-4-(((methyl-d3)amino)methyl)-1H-pyrrol-1-yl)sulfonyl)benzonitrile compound

The compound of Example 22-1 was prepared into the compound of Example 22, as the title compound, according to Preparation Example 2-3. (Yield: 12%)

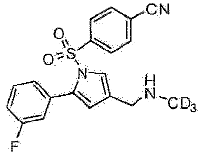

¹H NMR (500 MHz, CDCl₃) δ 7.66 - 7.61 (m, 2H), 7.51 - 7.45 (m, 2H), 7.36 - 7.33 (m, 1H), 7.32 - 7.27 (m, 1H), 7.09 (tdd, J = 8.4, 2.6, 0.9 Hz, 1H), 7.03 - 6.99 (m, 1H), 6.95 (ddd, J = 9.6, 2.5, 1.6 Hz, 1H), 6.23 (d, J = 1.9 Hz, 1H), 3.60 (s, 2H).

### Example 23. Preparation of N-((5-(3-fluorophenyl)-1-((4-(trifluoromethyl)phenyl)sulfonyl)-1H-pyrrol-3-yl)methyl)methan-d₃-amine (corresponding to Scheme 2)

### 23-1. Preparation of 5-(3-fluorophenyl)-1-((4-(trifluoromethyl)phenyl)sulfonyl)-1H-pyrrole-3-carbaldehyde compound

The compound of Example 23-1, as the title compound, was obtained according to Preparation Example 2-2. (Yield: 86%)

¹H NMR (500 MHz, CDCl₃) δ 9.89 (s, 1H), 8.12 (t, J = 2.3 Hz, 1H), 7.63 (d, J = 8.3 Hz, 2H), 7.50 (d, J = 8.2 Hz, 2H), 7.31 - 7.26 (m, 1H), 7.13 ( tdd, J = 8.4, 2.6, 1.0 Hz, 1H), 6.94 (ddd, J = 7.6, 1.5, 1.1 Hz, 1H), 6.87 (ddd, J = 9.4, 2.5, 1.6 Hz, 1H), 6.61 (d, J = 1.9 Hz, 1H).

### 23-2. Preparation of N-((5-(3-fluorophenyl)-1-((4-(trifluoromethyl)phenyl)sulfonyl)-1H-pyrrol-3-yl)methyl)methan-d₃-amine compound

The compound of Example 23-1 was prepared into the compound of Example 23, as the title compound, according to Preparation Example 2-3. (Yield: 35%)

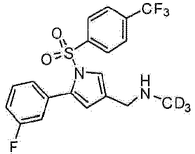

¹H NMR (500 MHz, CDCl₃) δ 7.61 (d, J = 8.3 Hz, 2H), 7.51 (d, J = 8.3 Hz, 2H), 7.43 - 7.38 (m, 1H), 7.31 - 7.26 (m , 1H), 7.08 (tdd, J = 8.4, 2.6, 0.9 Hz, 1H), 7.04 - 6.99 (m ,1H), 6.94 (ddd, J = 9.7, 2.5, 1.6 Hz, 1H), 6.25 (d, J = 1.9 Hz, 1H), 3.64 (d, J = 0.4 Hz, 2H).

### Example 24. Preparation of N-((5-(4-fluorophenyl)-1-((4-fluorophenyl)sulfonyl)-1H-pyrrol-3-yl)methyl)methan-d₃-amine (corresponding to Scheme 2)

### 24-1. Preparation of 5-(4-fluorophenyl)-1-((4-fluorophenyl)sulfonyl)-1H-pyrrole-3-carbaldehyde compound

The compound of Example 24-1, as the title compound, was obtained according to Preparation Example 2-2. (Yield: 52%)

¹H NMR (500 MHz, CD₃OD) δ 9.84 - 9.78 (s, 1H), 8.32 (d, J = 1.9 Hz, 1H), 7.46 (dd, J = 9.1, 4.9 Hz, 2H), 7.19 - 7.13 (m, 4H), 7.05 (t, J = 8.8 Hz, 2H), 6.54 (d, J = 1.8 Hz, 1H).

### 24-2. Preparation of N-((5-(4-fluorophenyl)-1-((4-fluorophenyl)sulfonyl)-1H-pyrrol-3-yl)methyl)methane-d₃-amine compound

The compound of Example 24-1 was prepared into the compound of Example 24, as the title compound, according to Preparation Example 2-3. (Yield: 50%)

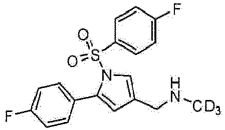

¹H NMR (500 MHz, CD₃OD) δ 7.63 (s, 1H), 7.55 - 7.51 (m, 2H), 7.33 - 7.29 (m, 2H), 7.26 (td, J = 6.8, 3.5 Hz, 2H), 7.18 - 7.14 (m, 2H), 6.37 (d, J = 1.9 Hz, 1H), 3.81 (s, 2H).

### Example 25. Preparation of N-((1-((4-bromophenyl)sulfonyl)-5-(4-fluorophenyl)-1H-pyrrol-3-yl)methyl)methan-d₃-amine (corresponding to Scheme 2)

### 25-1. Preparation of 1-((4-bromophenyl)sulfonyl)-5-(4-fluorophenyl)-1H-pyrrole-3-carbaldehyde compound

The compound of Example 25-1, as the title compound, was obtained according to Preparation Example 2-2. (Yield: 29%)

¹H NMR (500 MHz, CD₃OD) δ 9.85 (s, 1H), 8.40 (d, J = 1.8 Hz, 1H), 7.88 - 7.84 (m, 1H), 7.57 - 7.51 (m, 2H), 7.46 - 7.41 (m, 2H), 7.20 (td, J = 7.6, 1.0 Hz, 1H), 7.12 - 7.07 (m, 2H), 6.64 (d, J = 1.8 Hz, 1H).

### 25-2. Preparation of N-((1-((4-bromophenyl)sulfonyl)-5-(4-fluorophenyl)-1H-pyrrol-3-yl)methyl)methan-d₃-amine compound

The compound of Example 25-1 was prepared into the compound of Example 25, as the title compound, according to Preparation Example 2-3. (Yield: 27%)

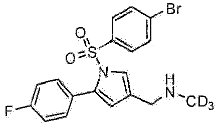

¹H NMR (500 MHz, CD₃OD) δ 7.61 (d, J = 2.0 Hz, 1H), 7.59 (dd, J = 3.0, 2.0 Hz, 2H), 7.27 - 7.23 (m, 2H), 7.20 - 7.16 (m, 2H), 7.06 (t, J = 8.8 Hz, 2H), 6.28 (d, J = 1.9 Hz, 1H), 3.89 (s, 2H).

### Example 26. Preparation of N-((1-((4-chlorophenyl)sulfonyl)-5-(4-fluorophenyl)-1H-pyrrol-3-yl)methyl)methan-d₃-amine (corresponding to Scheme 2)

### 26-1. Preparation of 1-((4-chlorophenyl)sulfonyl)-5-(4-fluorophenyl)-1H-pyrrole-3-carbaldehyde compound

The compound of Example 26-1, as the title compound, was obtained according to Preparation Example 2-2. (Yield: 33%)

¹H NMR (500 MHz, CDCl₃) δ 9.88 (s, 1H), 8.08 (d, J = 1.8 Hz, 1H), 7.36 - 7.31 (m, 2H), 7.28 - 7.26 (m, 1H), 7.25 (d, J = 2.4 Hz, 1H), 7.18 - 7.13 (m, 2H), 7.06 - 7.00 (m, 2H), 6.57 (d, J = 1.9 Hz, 1H).

### 26-2. Preparation of N-((1-((4-chlorophenyl)sulfonyl)-5-(4-fluorophenyl)-1H-pyrrol-3-yl)methyl)methan-d₃-amine compound

The compound of Example 26-1 was prepared into the compound of Example 26, as the title compound, according to Preparation Example 2-3. (Yield: 24%) 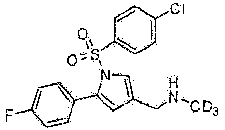 ¹H NMR (500 MHz, CD₃OD) δ 7.94 (dd, J = 10.9, 8.9 Hz, 1H), 7.63 (d, J = 1.5 Hz, 1H), 7.47 - 7.42 (m, 2H), 7.36 - 7.32 (m, 2H), 7.20 - 7.15 (m, 2H), 7.08 - 7.04 (m, 2H), 6.29 (d, J = 1.9 Hz, 1H), 3.96 (s, 2H).

### Example 27. Preparation of N-((5-(4-fluorophenyl)-1-((4-methoxyphenyl)sulfonyl)-1H-pyrrol-3-yl)methyl)methan-d₃-amine (corresponding to Scheme 2)

### 27-1. Preparation of 5-(4-fluorophenyl)-1-((4-methoxyphenyl)sulfonyl)-1H-pyrrole-3-carbaldehyde compound

The compound of Example 27-1, as the title compound, was obtained according to Preparation Example 2-2. (Yield: 66%)

¹H NMR (700 MHz, CDCl₃) δ 9.87 (s, 1H), 8.09 (d, J = 1.9 Hz, 1H), 7.26 (d, J = 2.1 Hz, 1H), 7.25 (d, J = 2.1 Hz, 1H), 7.16 (dd, J = 8.7, 5.4 Hz, 2H), 7.00 (t, J = 8.6 Hz, 2H), 6.80 - 6.76 (m, 2H), 6.54 (d, J = 1.9 Hz, 1H), 3.83 (s, 3H).

### 27-2. Preparation of N-((5-(4-fluorophenyl)-1-((4-methoxyphenyl)sulfonyl)-1H-pyrrol-3-yl)methyl)methan-d₃-amine compound

The compound of Example 27-1 was prepared into the compound of Example 27, as the title compound, according to Preparation Example 2-3. (Yield: 30%)

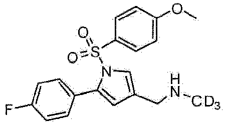

¹H NMR (500 MHz, CD₃OD) δ 7.87 (d, J = 9.1 Hz, 1H), 7.62 - 7.59 (m, 1H), 7.30 - 7.27 (m, 2H), 7.19 - 7.15 (m, 2H), 7.07 - 7.03 (m, 2H), 6.91 - 6.87 (m, 2H), 6.24 (d, J = 2.0 Hz, 1H), 3.94 (s, 2H), 3.81 (s, 3H).

### Example 28. Preparation of N-((5-(4-fluorophenyl)-1-tosyl-1H-pyrrol-3-yl)methyl)methan-d₃-amine (corresponding to Scheme 2)

### 28-1. Preparation of 5-(4-fluorophenyl)-1-tosyl-1H-pyrrole-3-carbaldehyde compound

The compound of Example 28-1, as the title compound, was obtained according to Preparation Example 2-2. (Yield: 42%)

¹H NMR (700 MHz, CDCl₃) δ 9.87 (s, 1H), 8.09 (d, J = 1.9 Hz, 1H), 7.22 (d, J = 8.4 Hz, 2H), 7.14 (ddd, J = 8.8, 3.3, 2.4 Hz, 4H), 6.99 (t, J = 8.7 Hz, 2H), 6.54 (d, J = 1.8 Hz, 1H), 2.39 (s, 3H).

### 28-2. Preparation of N-((5-(4-fluorophenyl)-1-tosyl-1H-pyrrol-3-yl)methyl)methan-d₃-amine compound

The compound of Example 28-1 was prepared into the compound of Example 28, as the title compound, according to Preparation Example 2-3. (Yield: 42%)

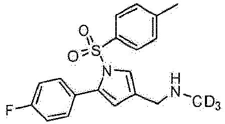

¹H NMR (500 MHz, CD₃OD) δ 7.83 (dd, J = 14.4, 8.4 Hz, 1H), 7.63 (dd, J = 1.3, 0.7 Hz, 1H), 7.25 - 7.20 (m, 4H), 7.17 - 7.13 (m, 2H), 7.06 - 7.01 (m, 2H), 6.26 (d, J = 2.0 Hz, 1H), 3.98 (s, 2H), 2.35 (s, 3H).

### Example 29. Preparation of N-((5-(4-fluorophenyl)-1-((4-(trifluoromethoxy)phenyl)sulfonyl)-1H-pyrrol-3-yl)methyl)methan-d₃-amine (corresponding to Scheme 2)

### 29-1. Preparation of 5-(4-fluorophenyl)-1-((4-(trifluoromethoxy)phenyl)sulfonyl)-1H-pyrrole-3-carbaldehyde compound

The compound of Example 29-1, as the title compound, was obtained according to Preparation Example 2-2. (Yield: 64%)

¹H NMR (700 MHz, CDCl₃) δ 9.89 (s, 1H), 8.10 (d, J = 1.9 Hz, 1H), 7.39 - 7.37 (m, 2H), 7.16 (d, J = 8.1 Hz, 2H), 7.15 - 7.12 (m, 2H), 7.02 - 6.98 (m, 2H), 6.58 (d, J = 1.8 Hz, 1H).

### 29-2. Preparation of N-((5-(4-fluorophenyl)-1-((4-(trifluoromethoxy)phenyl)sulfonyl)-1H-pyrrol-3-yl)methyl)methan-d₃-amine compound

The compound of Example 29-1 was prepared into the compound of Example 29, as the title compound, according to Preparation Example 2-3. (Yield: 20%) 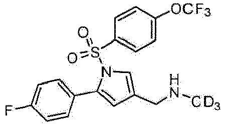 ¹H NMR (500 MHz, CD₃OD) δ 8.10 (d, J = 9.1 Hz, 1H), 7.63 - 7.60 (m, 1H), 7.52 - 7.50 (m, 2H), 7.35 (dd, J = 8.9, 0.9 Hz, 2H), 7.23 - 7.19 (m, 2H), 7.10 - 7.05 (m, 2H), 6.32 (d, J = 1.9 Hz, 1H), 3.86 (s, 2H).

### Example 30. Preparation of N-((5-(4-fluorophenyl)-1-((4-(trifluoromethyl)phenyl)sulfonyl)-1H-pyrrol-3-yl)methyl)methan-d₃-amine (corresponding to Scheme 2)

### 30-1. Preparation of 5-(4-fluorophenyl)-1-((4-(trifluoromethyl)phenyl)sulfonyl)-1H-pyrrole-3-carbaldehyde compound

The compound of Example 30-1, as the title compound, was obtained according to Preparation Example 2-2. (Yield: 63%)

¹H NMR (700 MHz, CDCl₃) δ 9.89 (s, 1H), 8.11 (d, J = 1.9 Hz, 1H), 7.63 (d, J = 8.3 Hz, 2H), 7.47 (d, J = 8.3 Hz, 2H), 7.14 (dd, J = 8.8, 5.3 Hz, 2H), 7.02 (t, J = 8.6 Hz, 2H), 6.59 (d, J = 1.8 Hz, 1H).

### 30-2. Preparation of N-((5-(4-fluorophenyl)-1-((4-(trifluoromethyl)phenyl)sulfonyl)-1H-pyrrol-3-yl)methyl)methan-d₃-amine compound

The compound of Example 30-1 was prepared into the compound of Example 30, as the title compound, according to Preparation Example 2-3. (Yield: 21%)

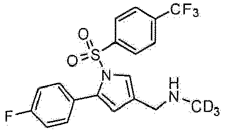

¹H NMR (500 MHz, CD₃OD) δ 7.78 (d, J = 8.3 Hz, 2H), 7.60 (d, J = 8.3 Hz, 2H), 7.57 (d, J = 1.3 Hz, 1H), 7.26 - 7.21 (m, 2H), 7.12 - 7.06 (m, 2H), 6.31 (d, J = 1.7 Hz, 1H), 3.75 (d, J = 9.5 Hz, 2H).

### Example 31. Preparation of 4-((2-(4-fluorophenyl)-4-(((methyl-d₃)amino)methyl)-1H-pyrrol-1-yl)sulfonyl)benzonitrile (corresponding to Scheme 2)

### 31-1. Preparation of 4-((2-(4-fluorophenyl)-4-formyl-1H-pyrrol-1-yl)sulfonyl)benzonitrile compound

The compound of Example 31-1, as the title compound, was obtained according to Preparation Example 2-2. (Yield: 57%)

¹H NMR (700 MHz, CDCl₃) δ 9.89 (s, 1H), 8.09 (d, J = 1.9 Hz, 1H), 7.68 - 7.63 (m, 2H), 7.46 - 7.43 (m, 2H), 7.15 (dd, J = 8.8, 5.3 Hz, 2H), 7.04 (t, J = 8.6 Hz, 2H), 6.60 (d, J = 1.8 Hz, 1H).

### 31-2. Preparation of 4-((2-(4-fluorophenyl)-4-(((methyl-d₃)amino)methyl)-1H-pyrrol-1-yl)sulfonyl)benzonitrile compound

The compound of Example 31-1 was prepared into the compound of Example 31, as the title compound, according to Preparation Example 2-3. (Yield: 12%)

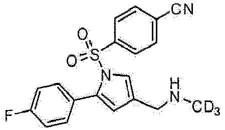

¹H NMR (500 MHz, CD₃OD) δ 7.84 (d, J = 8.6 Hz, 2H), 7.57 (s, 1H), 7.55 (d, J = 8.6 Hz, 2H), 7.25 - 7.20 (m, 2H), 7.10 (t, J = 8.8 Hz, 2H), 6.32 (d, J = 1.4 Hz, 1H), 3.78 (s, 2H).

### Example 32. Preparation of N-((5-(3-fluorophenyl)-1-((4-(trifluoromethoxy)phenyl)sulfonyl)-1H-pyrrol-3-yl)methyl)methan-d₃-amine (corresponding to Scheme 2)

### 32-1. Preparation of 5-(3-fluorophenyl)-1-((4-(trifluoromethoxy)phenyl)sulfonyl)-1H-pyrrole-3-carbaldehyde compound

The compound of Example 32-1, as the title compound, was obtained according to Preparation Example 2-2. (Yield: 26%)

¹H NMR (500 MHz, CDCl₃) δ 9.90 (s, 1H), 8.12 (d, J = 1.9 Hz, 1H), 7.45 - 7.40 (m, 2H), 7.32 - 7.27 (m, 1H), 7.18 (dd, J = 9.0, 0.9 Hz, 2H), 7.13 (tdd, J = 8.4, 2.6, 1.0 Hz, 1H), 6.96 (ddd, J = 7.6, 1.5, 1.1 Hz, 1H), 6.88 (ddd, J = 9.4, 2.5, 1.6 Hz, 1H), 6.62 (d, J = 1.9 Hz, 1H).

### 32-2. Preparation of N-((5-(3-fluorophenyl)-1-((4-(trifluoromethoxy)phenyl)sulfonyl)-1H-pyrrol-3-yl)methyl)methan-d₃-amine compound

The compound of Example 32-1 was prepared into the compound of Example 32, as the title compound, according to Preparation Example 2-3. (Yield: 40%)

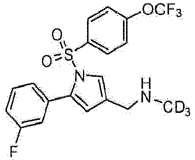

¹H NMR (500 MHz, CD₃OD) δ 7.63 - 7.58 (m, 1H), 7.53 - 7.45 (m ,2H), 7.36 - 7.27 (m, 3H), 7.13 (tdd, J = 8.6, 2.6, 0.9 Hz, 1H), 6.97 (ddd, J = 7.7, 1.4, 1.0 Hz, 1H), 6.91 (ddd, J = 9.8, 2.5, 1.6 Hz, 1H), 6.33 (d, J = 1.9 Hz, 1H), 3.85 (d, J = 5.5 Hz, 2H).

### Example 33. Preparation of N-((1-((4-fluorophenyl)sulfonyl)-5-(2-fluoropyridin-3-yl)-1H-pyrrol-3-yl)methyl)methan-d₃-amine (corresponding to Scheme 1)

### 33-1. Preparation of 1-((4-fluorophenyl)sulfonyl)-5-(2-fluoropyridin-3-yl)-1H-pyrrole-3-carbaldehyde compound

The compound of Example 33-1, as the title compound, was obtained according to Preparation Example 1-1. (Yield: 66%)

¹H NMR (700 MHz, CDCl₃) δ 9.89 (s, 1H), 8.31 (d, J = 3.7 Hz, 1H), 8.11 (d, J = 1.8 Hz, 1H), 7.75 (ddd, J = 9.2, 7.4, 1.9 Hz, 1H), 7.50 - 7.44 (m, 2H), 7.28 (ddd, J = 7.3, 4.9, 1.3 Hz, 1H), 7.16 - 7.09 (m, 2H), 6.72 (d, J = 1.7 Hz, 1H).

### 33-2. Preparation of N-((1-((4-fluorophenyl)sulfonyl)-5-(2-fluoropyridin-3-yl)-1H-pyrrol-3-yl)methyl)methan-d₃-amine compound

The compound of Example 33-1 was prepared into the compound of Example 33, as the title compound, according to Preparation Example 1-2. (Yield: 33%)

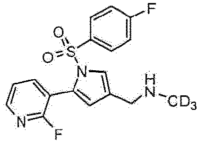

¹H NMR (500 MHz, CD₃OD) δ 8.27 (ddd, J = 4.9, 1.9, 0.8 Hz, 1H), 7.72 (ddd, J = 9.3, 7.4, 2.0 Hz, 1H), 7.64 - 7.60 (m, 1H), 7.58 - 7.52 (m, 2H), 7.36 (ddd, J = 7.3, 5.0, 1.6 Hz, 1H), 7.29 - 7.22 (m, 2H), 6.45 (d, J = 1.8 Hz, 1H), 3.81 (s, 2H).

### Example 34. Preparation of N-((1-((4-chlorophenyl)sulfonyl)-5-(2-fluoropyridin-3-yl)-1H-pyrrol-3-yl)methyl)methan-d₃-amine (corresponding to Scheme 1)

### 34-1. Preparation of 1-((4-chlorophenyl)sulfonyl)-5-(2-fluoropyridin-3-yl)-1H-pyrrole-3-carbaldehyde compound

The compound of Example 34-1, as the title compound, was obtained according to Preparation Example 1-1. (Yield: 86%)

¹H NMR (700 MHz, CDCl₃) δ 9.89 (s, 1H), 8.32 (ddd, J = 4.8, 1.9, 0.8 Hz, 1H), 8.10 (d, J = 1.8 Hz, 1H), 7.74 (ddd, J = 9.3, 7.4, 2.0 Hz, 1H), 7.45 - 7.41 (m, 2H), 7.40 - 7.36 (m, 2H), 7.28 (ddd, J = 7.3, 4.9, 1.5 Hz, 1H), 6.73 (d, J = 1.8 Hz, 1H).

### 34-2. Preparation of N-((1-((4-chlorophenyl)sulfonyl)-5-(2-fluoropyridin-3-yl)-1H-pyrrol-3-yl)methyl)methan-d₃-amine compound

The compound of Example 34-1 was prepared into the compound of Example 34, as the title compound, according to Preparation Example 1-2. (Yield: 37%)

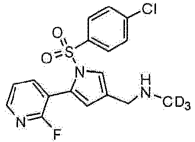

¹H NMR (700 MHz, CD₃OD) δ 8.32 - 8.23 (m, 1H), 7.75 (ddd, J = 9.3, 7.4, 1.9 Hz, 1H), 7.57 - 7.53 (m, 3H), 7.51 - 7.46 (m, 2H), 7.38 (ddd, J = 7.2, 5.0, 1.5 Hz, 1H), 6.45 (d, J = 1.8 Hz, 1H), 3.66 (s, 2H).

### Example 35. Preparation of N-((5-(2,4-difluorophenyl)-4-methoxy-1-(pyridin-3-ylsulfonyl)-1H-pyrrol-3-yl)methyl)methan-d₃-amine (corresponding to Scheme 3)

### 35-1. Preparation of methyl 5-(2,4-difluorophenyl)-4-methoxy-1-(pyridin-3-ylsulfonyl)-1H-pyrrole-3-carboxylate compound

The compound of Example 35-1, as the title compound, was obtained according to Preparation Example 3-1. (Yield: 75%)

¹H NMR (500 MHz, CDCl₃) δ 8.82 (dd, J = 4.8, 1.6 Hz, 1H), 8.59 (dd, J = 2.4, 0.5 Hz, 1H), 8.00 (s, 1H), 7.70 (ddd, J = 8.1, 2.4, 1.6 Hz, 1H), 7.38 (ddd, J = 8.2, 4.8, 0.8 Hz, 1H), 7.16 (td, J = 8.3, 6.4 Hz, 1H), 6.96 - 6.90 (m, 1H), 6.78 (td, J = 9.0, 2.5 Hz, 1H), 3.87 (s, 3H), 3.62 (s, 3H).

### 35-2. Preparation of 5-(2,4-difluorophenyl)-4-methoxy-1-(pyridin-3-ylsulfonyl)-1H-pyrrole-3-carbaldehyde compound

The compound of Example 35-1 was prepared into the compound of Example 35, as the title compound, according to Preparation Example 3-2. (Yield: 27%)

¹H NMR (500 MHz, CDCl₃) δ 9.90 (s, 1H), 8.84 (dd, J = 4.8, 1.4 Hz, 1H), 8.59 (d, J = 2.1 Hz, 1H), 8.01 (s, 1H), 7.70 (ddd, J = 8.1, 2.4, 1.6 Hz, 1H), 7.40 (ddd, J = 8.1, 4.8, 0.6 Hz, 1H), 7.23 - 7.17 (m, 1H), 6.97 - 6.92 (m, 1H), 6.77 (td, J = 8.9, 2.5 Hz, 1H), 3.63 (s, 3H).

### 35-3. Preparation of N-((5-(2,4-difluorophenyl)-4-methoxy-1-(pyridin-3-ylsulfonyl)-1H-pyrrol-3-yl)methyl)methan-d₃-amine compound

The compound of Example 35-2 was prepared into the compound of Example 35, as the title compound, according to Preparation Example 3-1. (Yield: 31%)

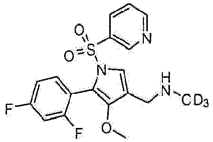

¹H NMR (500 MHz, CDCl₃) δ 8.77 (dd, J = 4.8, 1.6 Hz, 1H), 8.59 (dd, J = 2.4, 0.6 Hz, 1H), 7.73 (ddd, J = 8.1, 2.4, 1.6 Hz, 1H), 7.40 - 7.34 (m, 2H), 7.17 (td, J = 8.4, 6.4 Hz, 1H), 6.94 - 6.87 (m, 1H), 6.80 (td, J = 9.0, 2.5 Hz, 1H), 3.68 (d, J = 1.2 Hz, 2H), 3.45 (s, 3H).

### Example 36. Preparation of N-((5-(2-fluorophenyl)-4-methoxy-1-(pyridin-3-ylsulfonyl)-1H-pyrrol-3-yl)methyl)methan-d₃-amine (corresponding to Scheme 3)

### 36-1. Preparation of methyl 5-(2-fluorophenyl)-4-methoxy-1-(pyridin-3-ylsulfonyl)-1H-pyrrole-3-carboxylate compound

The compound of Example 36-1, as the title compound, was obtained according to Preparation Example 3-1. (Yield: 47%)

¹H NMR (500 MHz, CDCl₃) δ 8.80 (dd, J = 4.8, 1.5 Hz, 1H), 8.54 (d, J = 2.4 Hz, 1H), 8.01 (s, 1H), 7.69 - 7.65 (m, 1H), 7.48 - 7.43 (m, 1H), 7.35 (dd, J = 8.1, 4.8 Hz, 1H), 7.20 - 7.15 (m, 2H), 7.01 (t, J = 8.8 Hz, 1H), 3.88 (s, 3H), 3.61 (s, 3H).

### 36-2. Preparation of 5-(2-fluorophenyl)-4-methoxy-1-(pyridin-3-ylsulfonyl)-1H-pyrrole-3-carbaldehyde compound

The compound of Example 36-1 was prepared into the compound of Example 36, as the title compound, according to Preparation Example 3-2. (Yield: 32%)

¹H NMR (500 MHz, CDCl₃) δ 9.91 (s, 1H), 8.82 (dd, J = 4.8, 1.6 Hz, 1H), 8.52 (dd, J = 2.4, 0.6 Hz, 1H), 8.02 (s, 1H), 7.67 (ddd, J = 8.1, 2.4, 1.6 Hz, 1H), 7.50 - 7.44 (m, 1H), 7.37 (ddd, J = 8.1, 4.8, 0.8 Hz, 1H), 7.24 - 7.16 (m, 2H), 7.02 - 6.97 (m, 1H), 3.61 (s, 3H).

### 36-3. Preparation of N-((5-(2-fluorophenyl)-4-methoxy-1-(pyridin-3-ylsulfonyl)-1H-pyrrol-3-yl)methyl)methan-d₃-amine compound

The compound of Example 36-2 was prepared into the compound of Example 36, as the title compound, according to Preparation Example 3-1. (Yield: 31%)

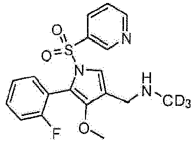

¹H NMR (500 MHz, CDCl₃) δ 8.76 (dd, J = 4.8, 1.2 Hz, 1H), 8.53 (d, J = 2.1 Hz, 1H), 7.81 - 7.75 (m, 1H), 7.58 (s, 1H), 7.49 - 7.39 (m, 2H), 7.21 - 7.12 (m, 2H), 7.02 (t, J = 8.8 Hz, 1H), 3.93 (s, 2H), 3.42 (s, 3H).

### Example 37. Preparation of N-((5-(2,4-difluorophenyl)-4-methoxy-1-((6-methoxypyridin-3-yl)sulfonyl)-1H-pyrrol-3-yl)methyl) methane-d₃-amine (corresponding to Scheme 3)

### 37-1. Preparation of methyl 5-(2,4-difluorophenyl)-4-methoxy-1-((6-methoxypyridin-3-yl)sulfonyl)-1H-pyrrole-3-carboxylate compound

The compound of Example 37-1, as the title compound, was obtained according to Preparation Example 3-1. (Yield: 78%)

¹H NMR (500 MHz, CDCl₃) δ 8.14 (dd, J = 2.6, 0.5 Hz, 1H), 7.97 (s, 1H), 7.50 (dd, J = 8.9, 2.7 Hz, 1H), 7.19 (td, J = 8.3, 6.4 Hz, 1H), 6.96 - 6.90 (m, 1H), 6.79 (td, J = 9.0, 2.5 Hz, 1H), 6.71 (dd, J = 8.9, 0.6 Hz, 1H), 3.99 (s, 3H), 3.87 (s, 3H), 3.62 (s, 3H).

### 37-2. Preparation of 5-(2,4-difluorophenyl)-4-methoxy-1-((6-methoxypyridin-3-yl)sulfonyl)-1H-pyrrole-3-carbaldehyde compound

The compound of Example 37-1 was prepared into the compound of Example 37, as the title compound, according to Preparation Example 3-2. (Yield: 30%)

¹H NMR (500 MHz, CDCl₃) δ 9.88 (s, 1H), 8.13 (dd, J = 2.7, 0.6 Hz, 1H), 7.98 (s, 1H), 7.49 (dd, J = 8.9, 2.7 Hz, 1H), 7.23 (td, J = 8.3, 6.4 Hz, 1H), 6.97 - 6.92 (m, 1H), 6.78 (td, J = 9.0, 2.5 Hz, 1H), 6.72 (dd, J = 8.9, 0.6 Hz, 1H), 3.99 (s, 3H), 3.64 (s, 3H).

### 37-3. Preparation of N-((5-(2,4-difluorophenyl)-4-methoxy-1-((6-methoxypyridin-3-yl)sulfonyl)-1H-pyrrol-3-yl)methyl) methane-d₃-amine compound

The compound of Example 37-2 was prepared into the compound of Example 37, as the title compound, according to Preparation Example 3-1. (Yield: 16%)

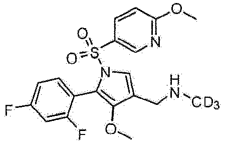

¹H NMR (500 MHz, CDCl₃) δ 8.15 (dd, J = 2.6, 0.4 Hz, 1H), 7.53 (dd, J = 8.9, 2.6 Hz, 1H), 7.32 (s, 1H), 7.20 (td, J = 8.4, 6.4 Hz, 1H), 6.93 - 6.87 (m, 1H), 6.80 (td, J = 9.0, 2.5 Hz, 1H), 6.68 (dd, J = 8.9, 0.6 Hz, 1H), 3.97 (s, 3H), 3.66 (d, J = 2.9 Hz, 2H), 3.45 (s, 3H).

### Example 38. Preparation of N-((4-methoxy-1-((6-(trifluoromethyl)pyridin-3-yl)sulfonyl)-5-(2,4,6-trifluorophenyl)-1H-pyrrole-3-yl)methyl)methane-d₃-amine (corresponding to Scheme 3)

### 38-1. Preparation of methyl 4-methoxy-1-((6-(trifluoromethyl)pyridin-3-yl)sulfonyl)-5-(2,4,6-trifluorophenyl)-1H-pyrrole-3-carboxylate compound

The compound of Example 38-1, as the title compound, was obtained according to Preparation Example 3-1. (Yield: 48%)

¹H NMR (500 MHz, CDCl₃) δ 8.78 (d, J = 2.2 Hz, 1H), 8.04 (s, 1H), 8.00 (dd, J = 8.2, 2.2 Hz, 1H), 7.80 (dd, J = 8.3, 0.6 Hz, 1H), 6.73 (dd, J = 8.5, 7.1 Hz, 2H), 3.88 (s, 3H), 3.68 (s, 3H).

### 38-2. Preparation of 4-methoxy-1-((6-(trifluoromethyl)pyridin-3-yl)sulfonyl)-5-(2,4,6-trifluorophenyl)-1H-pyrrole-3-carbaldehyde compound

The compound of Example 38-1 was prepared into the compound of Example 38, as the title compound, according to Preparation Example 3-2. (Yield: 31%)

¹H NMR (500 MHz, CDCl₃) δ 9.93 (s, 1H), 8.78 (d, J = 2.2 Hz, 1H), 8.06 (s, 1H), 8.03 (dd, J = 8.3, 2.2 Hz, 1H), 7.88 (s, 1H), 7.84 (dd, J = 8.3, 0.5 Hz, 1H), 6.46 (d, J = 5.8, 1H), 3.70 (s, 3H).

### 38-3. Preparation of N-((4-methoxy-1-((6-(trifluoromethyl)pyridin-3-yl)sulfonyl)-5-(2,4,6-trifluorophenyl)-1H-pyrrole-3-yl)methyl)methane-d₃-amine compound

The compound of Example 38-2 was prepared into the compound of Example 38, as the title compound, according to Preparation Example 3-1. (Yield: 14%)

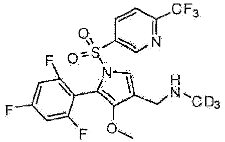

¹H NMR (500 MHz, CDCl₃) δ 8.76 (s, 1H), 8.01 (d, J = 8.1 Hz, 1H), 7.77 (d, J = 8.3 Hz, 1H), 7.45 (d, J = 24.5 Hz, 1H), 6.72 (t, J = 7.8 Hz, 2H), 3.64 (d, J = 31.1 Hz, 2H), 3.53 (s, 3H).

### Example 39. Preparation of N-((4-methoxy-1-(pyridin-3-ylsulfonyl)-5-(2,4,6-trifluorophenyl)-1H-pyrrol-3-yl)methyl)methan-d₃-amine (corresponding to Scheme 3)

### 39-1. Preparation of methyl 4-methoxy-1-(pyridin-3-ylsulfonyl)-5-(2,4,6-trifluorophenyl)-1H-pyrrole-3-carboxylate compound

The compound of Example 39-1, as the title compound, was obtained according to Preparation Example 3-1. (Yield: 94%)

¹H NMR (300 MHz, DMSO-d₆) δ 8.93 (dd, J = 4.8, 1.5 Hz, 1H), 8.77 - 8.67 (m, 1H), 8.19 (d, J = 1.2 Hz, 1H), 8.10 (ddd, J = 8.2, 2.5, 1.5 Hz, 1H), 7.66 (ddd, J = 8.2, 4.8, 0.8 Hz, 1H), 7.29 (dd, J = 9.2, 7.5 Hz, 2H), 3.79 (s, 3H), 3.54 (s, 3H).

### 39-2. Preparation of 4-methoxy-1-(pyridin-3-ylsulfonyl)-5-(2,4,6-trifluorophenyl)-1H-pyrrole-3-carbaldehyde compound

The compound of Example 39-1 was prepared into the compound of Example 39, as the title compound, according to Preparation Example 3-2. (Yield: 26%)

¹H NMR (500 MHz, CDCl₃) δ 9.90 (s, 1H), 8.88 (dd, J = 4.8, 1.6 Hz, 1H), 8.68 (d, J = 2.1 Hz, 1H), 8.06 (s, 1H), 7.80 (ddd, J = 8.2, 2.4, 1.6 Hz, 1H), 7.45 (ddd, J = 8.2, 4.8, 0.7 Hz, 1H), 6.72 (dd, J = 8.5, 6.9 Hz, 2H), 3.68 (d, J = 5.3 Hz, 3H).

### 39-3. Preparation of N-((4-methoxy-1-(pyridin-3-ylsulfonyl)-5-(2,4,6-trifluorophenyl)-1H-pyrrol-3-yl)methyl)methan-d₃-amine compound

The compound of Example 39-2 was prepared into the compound of Example 39, as the title compound, according to Preparation Example 3-1. (Yield: 40%)

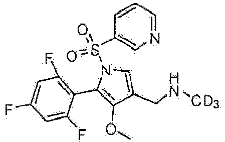

¹H NMR (500 MHz, CDCl₃) δ 8.79 (d, J = 4.8, 1.6 Hz, 1H), 8.66 (dd, J = 2.3, 0.4 Hz, 1H), 7.77 (ddd, J = 8.1, 2.4, 1.6 Hz, 1H), 7.38 (ddd, J = 8.1, 4.9, 0.8 Hz, 1H), 7.35 (s, 1H), 6.69 (dd, J = 8.6, 7.0 Hz, 2H), 3.61 (d, J = 0.8 Hz, 2H), 3.51 (s, 3H).

### Example 40. Preparation of N-((4-methoxy-1-((6-methoxypyridin-3-yl)sulfonyl)-5-(2,4,6-trifluorophenyl)-1H-pyrrol-3-yl)methyl)methane-d₃-amine (corresponding to Scheme 3)

### 40-1. Preparation of methyl 4-methoxy-1-((6-methoxypyridin-3-yl)sulfonyl)-5-(2,4,6-trifluorophenyl)-1H-pyrrole-3-carboxylate compound

The compound of Example 40-1, as the title compound, was obtained according to Preparation Example 3-1. (Yield: 48%)

¹H NMR (500 MHz, CDCl₃) δ 8.27 - 8.20 (m, 1H), 8.00 (s, 1H), 7.58 (dd, J = 8.9, 2.6 Hz, 1H), 6.74 (dd, J = 8.9, 0.6 Hz, 1H), 6.70 (dd, J = 8.6, 6.9 Hz, 2H), 4.00 (s, 3H), 3.86 (s, 3H), 3.67 (s, 3H).

### 40-2. Preparation of 4-methoxy-1-((6-methoxypyridin-3-yl)sulfonyl)-5-(2,4,6-trifluorophenyl)-1H-pyrrole-3-carbaldehyde compound

The compound of Example 40-1 was prepared into the compound of Example 40, as the title compound, according to Preparation Example 3-2. (Yield: 42%)

¹H NMR (500 MHz, CDCl₃) δ 9.88 (s, 1H), 8.24 (d, J = 2.5 Hz, 1H), 8.01 (s, 1H), 7.59 (dd, J = 8.9, 2.7 Hz, 1H), 6.77 (dd, J = 8.9, 0.5 Hz, 1H), 6.72 (dd, J = 8.6, 6.8 Hz, 2H), 4.01 (s. 3H), 3.69 (s, 3H).

### 40-3. Preparation of N-((4-methoxy-1-((6-methoxypyridin-3-yl)sulfonyl)-5-(2,4,6-trifluorophenyl)-1H-pyrrol-3-yl)methyl)methane-d₃-amine compound

The compound of Example 40-2 was prepared into the compound of Example 40, as the title compound, according to Preparation Example 3-1. (Yield: 39%)

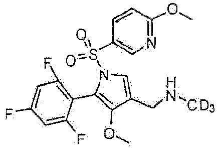

¹H NMR (500 MHz, CDCl₃) δ 8.23 (d, J = 2.4 Hz, 1H), 7.59 (dd, J = 8.8, 2.5 Hz, 1H), 7.33 (s, 1H), 6.74 - 6.60 (m, 3H), 3.98 (s, 3H), 3.64 (d, J = 22.5 Hz, 2H), 3.52 (s, 3H).

### Example 41. Preparation of N-((5-(2-fluorophenyl)-1-((4-fluorophenyl)sulfonyl)-1H-pyrrol-3-yl)methyl)methane-d₃-amine fumarate

The compound of Example 7 was suspended in methanol. The reaction mixture was added with fumaric acid (1 eq) and stirred under reflux for 60 minutes. It was cooled to room temperature and concentrated under reduced pressure. The concentrated residue was added with dimethyl ether, stirred for 5 minutes, and filtered to obtain the compound of Example 41 as the title compound. (Yield: 85%)

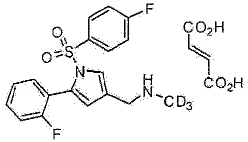

¹H NMR (700 MHz, DMSO-d₆) δ 7.74 (d, J = 1.5 Hz, 1H), 7.54 - 7.50 (m, 3H), 7.42 (t, J = 8.7 Hz, 2H), 7.22 (q, J = 8.2 Hz, 2H), 7.08 (td, J = 7.6, 1.5 Hz, 1H), 6.54 (s, 2H), 6.46 (d, J = 1.8 Hz, 1H), 3.96 (s, 2H).

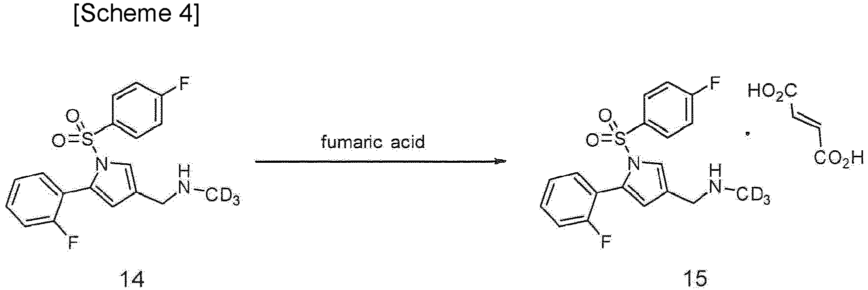

In Scheme 4, Step 1 is a step of preparing the compound represented by formula 14 into the compound of formula 15, which is a reaction of using an organic solvent such as isopropyl alcohol and methanol, and fumaric acid to prepare a fumarate salt.

### Example 42. Preparation of 5-(3-fluorophenyl)-1H-pyrrole-3-carbaldehyde

The compound of Example 42, as the title compound, was obtained by using 3-fluorophenylboronic acid according to Preparation Example 2-1. (Yield: 60%)

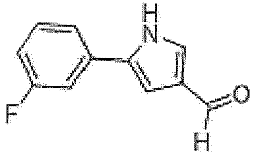

¹H NMR (300 MHz, DMSO-d₆) δ 9.71 (s, 1H), 8.23 (s, 1H), 7.79 - 7.75 (m, 1H), 7.59 - 7.50 (m, 2H), 7.42 (dd, J = 14.3, 7.4 Hz, 2H), 7.08 (dd, J = 5.0, 3.8 Hz, 1H).

### Example 43. Preparation of 5-(4-fluorophenyl)-1H-pyrrole-3-carbaldehyde

The compound of Example 43, as the title compound, was obtained by using 4-fluorophenylboronic acid according to Preparation Example 2-1. (Yield: 32%)

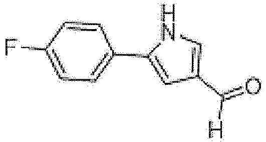

¹H NMR (500 MHz, CD₃OD) δ 9.66 - 9.64 (m, 1H), 7.63 - 7.58 (m, 3H), 7.14 - 7.10 (m, 2H), 6.83 (d, J = 1.6 Hz, 1H).

### Example 44. Preparation of 5-(2,4-difluorophenyl)-1H-pyrrole-3-carbaldehyde

The compound of Example 44, as the title compound, was obtained by using 2,4-fluorophenylboronic acid according to Preparation Example 2-1. (Yield: 58%)

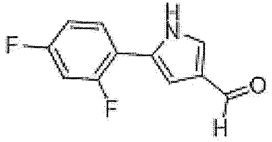

¹H NMR (500 MHz, CDCl₃) δ 9.85 (d, J = 6.5 Hz, 1H), 9.38 (s, 1H), 7.61 (td, J = 8.9, 6.2 Hz, 1H), 7.53 (dd, J = 3.1, 1.6 Hz, 1H), 7.02 - 6.87 (m, 3H).

### Control Drug 1. Vonoprazan

Vonoprazan (N-((5-(2-fluorophenyl)-1-(pyridin-3-ylsulfonyl)-1H-pyrrol-3-yl)methyl)methanamine), which is a commercial formulation of Potassium-Competitive Acid Blocker (P-CAB), was purchased from GLPBio (China).

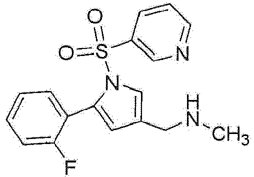

### Control Drug 2. Esomeprazole

Esomeprazole (6-methoxy-2-[(S)-(4-methoxy-3,5-dimethylpyridin-2-yl)methylsulfinyl]-1H-benzimidazole), which is a commercial Proton Pump Inhibitor (PPI) agent, was purchased from GLPBio (China).

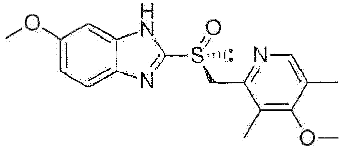

### Control Drug 3. Cisplatin

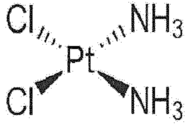

Cisplatin (diamminedichloridoplatinum(II)), which is used as an anticancer drug, was purchased from Glentham life sciences (UK).

### Comparative Example 1. Preparation of N-(5-(2-fluorophenyl)-1-((4-fluorophenyl)sulfonyl)-1H-pyrrol-3-ylyl)-N-methylmethanamine - deuterium-unsubstituted compound of Example 7

160 mg of 5-(2-fluorophenyl)-1-((4-fluorophenyl)sulfonyl)-1H-pyrrole-3-carbaldehyde of Example 7-1 was suspended in 3 ml of tetrahydrofuran and 7 ml of methanol. The reaction mixture was added with 186 mg (6 eq) of methylamine hydrochloride and 87 mg (3 eq) of sodium cyanoborohydride, and stirred at 20±5°C for 16 hours. The reaction mixture was added with 10 ml of purified water, 10 ml of aqueous sodium bicarbonate solution, and 10 ml of ethyl acetate to extract the organic layer. It was dried over sodium sulfate and filtered, and the filtrate was concentrated under reduced pressure. The concentrated residue was subjected to column chromatography in methylene chloride:methanol=10:1 to obtain 57 mg of the title compound. (Yield: 34%)

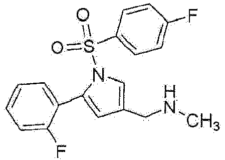

¹H NMR (300 MHz, CDCl₃) δ 7.41 - 7.29 (m, 4H), 7.16 - 7.05 (m, 2H), 7.05 - 6.99 (m, 3H), 6.23 (d, J = 1.8 Hz, 1H), 3.61 (s, 2H), 3.34 (s, 3H).

### Comparative Example 2. Preparation of N-((5-(2,4-difluorophenyl)-4-methoxy-1-(pyridin-3-ylsulfonyl)-1H-pyrrol-3-yl)methyl)methan-d₃-amine - deuterium unsubstituted compound of Example 35

530 mg of 5-(2,4-difluorophenyl)-4-methoxy-1-(pyridin-3-ylsulfonyl)-1H-pyrrole-3-carbaldehyde of Example 35-2 suspended in tetrahydrofuran 15. ml and 35 ml of methanol. The reaction mixture was added with 567 mg (6 eq) of methylamine hydrochloride and 264 mg (3 eq) of Sodium cyanoborohydride, and stirred at 20±5°C for 16 hours. The reaction mixture was added with 20 ml of sodium bicarbonate aqueous solution and 20 ml of ethyl acetate to extract the organic layer. It was dried over sodium sulfate and filtered, and the filtrate was concentrated under reduced pressure. The concentrated residue was subjected to column chromatography in methylene chloride:methanol=10:1 to obtain 184 mg of the title compound. (Yield: 35%)

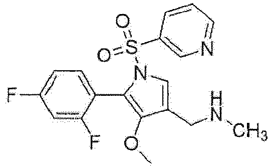

¹H NMR (500 MHz, CDCl₃) δ 8.78 (dd, J = 4.8, 1.6 Hz, 1H), 8.63 - 8.56 (m, 1H), 7.71 (ddd, J = 8.1, 2.3, 1.7 Hz, 1H), 7.36 (ddd, J = 8.2, 4.8, 0.7 Hz, 1H), 7.32 (s, 1H), 7.17 (td, J = 8.4, 6.4 Hz, 1H), 6.95 - 6.87 (m, 1H), 6.80 (td, J = 9.0, 2.5 Hz, 1H), 3.66 - 3.58 (m, 2H), 3.45 (s, 3H), 2.47 (s, 3H).

### <Test Examples>

### Test Example 1. inhibitory activity on proton pump (H⁺/K⁺-ATPase) activity

The compounds indicated in the above examples were tested for their inhibitory activity on proton pump (H⁺/K⁺-ATPase) activity as follows by applying the method disclosed in the literature. According to the methods of Nobuyuki Takahashi and Yukinori Take [J Pharmacol Exp Ther. 2018 Feb;364(2):275-286 ], the gastric mucosal microsome fraction was isolated from the porcine stomach. The stomach isolated from the pig was washed first with tap water, only the fundus was cut, and then washed (a second time) with cold PBS. The gastric mucosa was separated by scraping, homogenized with 250 mM sucrose solution, and centrifuged at 20,000 g at 4°C for 30 minutes. Only the supernatant was centrifuged at 115,000 g at 4°C for 30 minutes, and the precipitate was suspended in 250 mM sucrose solution, added to cover with 7% Ficoll solution, and centrifuged at 135,000 g at 4°C for 60 minutes. The microsome fraction formed at the interface was recovered and suspended in 250 mM sucrose solution, and centrifuged at 135,000 g, 4°C for 60 minutes, and the precipitate was dissolved in 0.9% NaCl. The isolated gastric mucosal microsome fraction was quantified by using the Bicinchoninic Acid (BCA) kit (Thermo).

60 µl of enzyme reaction solution (a test compound at a given concentration, 3 g of gastric mucosal microsomes, 3 mM of MgSO₄, 5 mM of KCI, 40 mM of Tris-HCl pH 6.4, and 0.5 mM of adenosine triphosphate) was added to each well of a 96-well plate and reacted at 37°C for 60 minutes, and 30 µl of 10% SDS was added to stop the enzyme reaction. 200 µl of detection solution (in which 10% L-ascorbic acid with pH 5.0 was mixed with 35 mM of ammonium molybdate tetrahydrate/15 mM of zinc acetate with pH 5.0 at the ratio of 4:1) was added and reacted at 37°C for 30 minutes, and the absorbance value was measured at 750 nm by using a microplate reader (MULTISKAN GO, Thermo). The inhibition rate of proton pump activity was expressed as IC₅₀, which is 50% inhibition of enzyme activity, by using GraphPad Prism 5. The results are shown in Table 1 below. In Table 1 below, an IC₅₀ value of 100 nM or less is indicated by +++, an IC₅₀ value of 101 to 200 nM is indicated by ++, and an IC₅₀ value of above 200 nM is indicated by +.

**[Table 1]**

| **Compound** | **Proton pump inhibitory activity (IC₅₀, nM)** |
|---|---|
| Control Drug 1 (Vonoprazan) | 42 (+++) |
| Example 2 | 68 (+++) |
| Example 3 | 48 (+++) |
| Example 4 | 90 (+++) |
| Example 5 | 36 (+++) |
| Example 6 | 26 (+++) |
| Example 7 | 25 (+++) |
| Example 8 | 51 (+++) |
| Example 10 | 60 (+++) |
| Example 11 | 76 (+++) |
| Example 16 | 82 (+++) |
| Example 17 | 84 (+++) |
| Example 37 | 88 (+++) |
| Example 39 | 38 (+++) |
| Example 40 | 64 (+++) |

As shown in Table 1 above, it is confirmed that the compounds of the working examples of the present invention showed excellent inhibitory activity on proton pump activity.

### Test Example 2. Confirmation of the effect of inhibiting the proliferation of human gastric cancer cells (AGS cells)

In the case of cancer cells, hydrogen ions accumulate in the cytoplasm due to the Warburg effect, leading to a decrease in intracellular pH. When the inside of the cell (Cytoplasm) becomes acidic, the activity and function of a protein are inhibited. Since the cell dies in severe cases, cancer cells actively release lactic acid or hydrogen ions from the cells. Therefore, potassium-competitive acid blocker (P-CAB) may inhibit the growth of cancer cells. [Cancers. 2020 Mar;12(3):640]

To examine the effect of inhibiting the proliferation of the compounds indicated in the examples of the present invention on human gastric cancer cells (AGS Cells), MTS Assay experiments were *in vitro* performed. AGS cells were cultured in a 5% CO₂ incubator at 37°C by using RPMI 1640 medium containing 1% penicillin-streptomycin and 10% fetal bovine serum. The cells in culture were separated with using 0.25% Trypsin-0.02% EDTA every 3 to 4 days and subcultured.

For the experiment, the cultured cells were treated with 0.25% Trypsin-0.02% EDTA solution, the suspended cells were centrifuged at 1,200 rpm for 5 minutes, the supernatant was discarded, a culture medium was added thereto, and a cell suspension was made with the culture medium. Using a hemocytometer, 10,000 cells per well were dispensed into 96-well plates and cultured. After 24 hours, when the cells attached to the plate, the culture medium was discarded, and the cells were treated with new culture media prepared by concentration (test concentrations: 500 µm, 250 µm, 125 µm, 63 µm, 31 µm, 16 µm, 8 µm, 4 µm, and 2 µm) and cultured in a CO₂ incubator for 48 hours. After treating with an MTS solution, the cells were incubated in a CO₂ incubator for 1 hour, and the absorbance was measured at 490 nm by using a microplate reader device. The cell proliferation inhibition rate was expressed as IC₅₀, which is 50% inhibition of cell proliferation, using GraphPad Prism 5.

The results are shown in Table 2 below. In Table 2 below, the IC₅₀ value is indicated as "+++" for 0 to 50 µM, "++" for 51 to 100 µM, and "+" for 101 to 400 µM.

**[Table 2]**

| **Compound** | **Proliferation inhibitory effect of gastric cancer cells (IC₅₀, µM)** |
|---|---|
| Control Drug 2 (Esomeprazole) | 194.4 (+) |
| Control Drug 3 (Cisplatin) | 19.2 (+++) |
| Example 2 | 17.5 (+++) |
| Example 3 | 3.5 (+++) |
| Example 4 | 32.7 (+++) |
| Example 6 | 21.7 (+++) |
| Example 7 | 35.3 (+++) |
| Example 8 | 12.2 (+++) |
| Example 13 | 14.2 (+++) |
| Example 22 | 22.6 (+++) |
| Example 24 | 41.5 (+++) |
| Example 25 | 24.8 (+++) |
| Example 26 | 13.2 (+++) |

As shown in Table 2 above, it was confirmed that some compounds in the Working examples of the present invention exhibited a significantly higher proliferation inhibitory effect on gastric cancer cells than that of esomeprazole, a commercial PPI agent, demonstrated a proliferation inhibition effect on gastric cancer cells equivalent to that of cisplatin used in gastric cancer treatment. From these results, it was found that the compounds of the present invention have excellent effects of preventing, improving, or treating gastric cancer, as well as excellent proton pump inhibitory effects.

### Test Example 3. Evaluation of anticancer efficacy in xenograft mouse model

This study was conducted to evaluate the anticancer efficacy of the compound of Example 15 of the present invention in a xenograft tumor model. Human gastric cancer cells (MKN-45 Cell) were purchased from the Korea Cell Line Bank and cultured in a 5% CO₂ incubator at 37°C by using an RPMI 1640 medium containing 1% penicillin -streptomycin and 10% fetal bovine serum. The cultured cells were separated using 0.25% Trypsin-0.02% EDTA every 3 to 4 days and then subcultured. Cells. Four-week-old female nude mice were purchased, and after a one-week acclimatization period in a laboratory environment, the nude mice that reached 5 weeks of age were used in the experiment. MKN-45 cells were made into a single cell suspension by using Matrigel and DPBS, and implanted subcutaneously into nude mice at a concentration of 1 x 10⁷ cells per mouse. One week after MKN-45 cell transplantation, experimental animals with tumors with a specific size were selected and divided into 3 groups with 10 animals per group. The compound of Example 7 was administered orally at administration concentrations of 10 mg/kg and 30 mg/kg, and the experiment was conducted for 12 days. The experimental conditions are shown in Table 3 below.

**[Table 3]**

| **Groups** | | **Concentration of admin. (mg/kg)** | **Administered substance** | **Number of animals** | **Route of admin.** | **Number of admin.** | **Time period of admin.** | **Gender** |
|---|---|---|---|---|---|---|---|---|
| G1 | Vehicle group | - | PEG400:0.5% MC (2:8) | 10 | Oral | Once/ day | 12 days | Female |
| G2 | Admini stration group 1 | 10 | Example 7 | 10 | Oral | | | |
| G3 | Admini stration group 2 | 30 | Example 7 | 10 | Oral | | | |

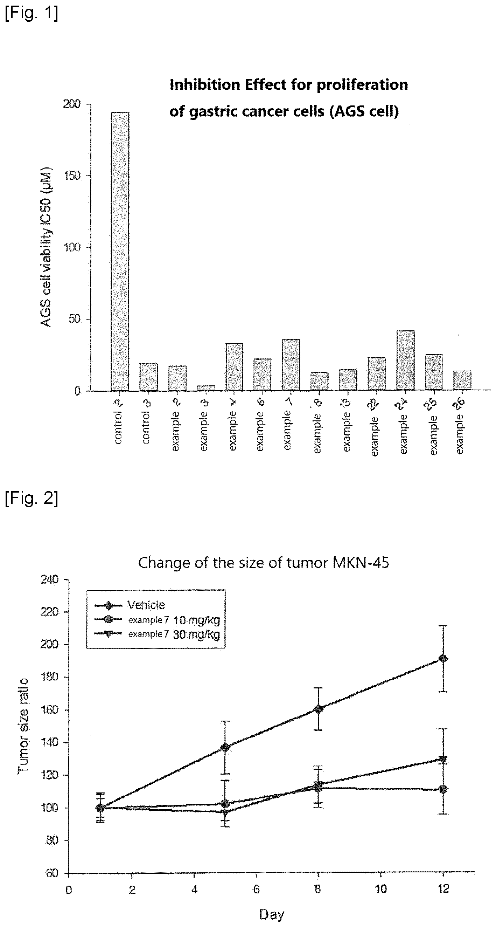

The size of the tumor was calculated using the formula 'short axis x short axis x long axis/2,' and the results of the changes in tumor size change are illustrated in Fig. 2. The results on Day 12 showed that, when 10 mg/kg of Example 7 was administered, the tumor size was reduced by 42% as compared to the Controal group (vehicle).

### Test Example 4. Pharmacokinetic and pharmacodynamic evaluation in beagles

### Animal models

A total of 18 male beagle dogs (Marshall Beagle, Beijing) with an average weight of 9 to 11 kg were obtained, acclimatized, and distributed into a total of 6 groups, 3 dogs per test group. During the entire experiment, all beagle dogs were supplied with standardized solid food and water. The breeding room was kept under controlled and monitored conditions, while maintaining a temperature of 18 °C to 26 °C, a humidity of 40% to 70%, and a light-dark cycle of 12 hours-12 hours.

The test group consisted of beagle dogs that were orally administered the vehicle as the vehicle group, beagle dogs that were orally administered control drug 1 (vonoprazan) as the comparison group, and administration group was beagle dogs orally administered with Example 7. The classification of the test groups and the dosage of the administered substances are shown in Table 4 below.

**[Table 4]**

| **Groups** | | **Numb er of anima ls** | **Administered substance** | **Route of admin.** | **Concentration of samples (mg/mL** | **Dosage (mg/kg)** |
|---|---|---|---|---|---|---|
| G1 | Vehicle Group | 3 | 0.5% methylcellulose | Oral, a single dose | - | 0 |
| G2 | Administration | 3 | Example 7 | Oral, | 0.05 | 0.1 |
| | Group 1 | | | a single dose | | |
| G3 | Administration Group 2 | 3 | Example 7 | Oral, a single dose | 0.15 | 0.3 |
| G4 | Administration Group 3 | 3 | Example 7 | Oral, a single dose | 0.5 | 1 |
| G5 | Administration Group 4 | 3 | Example 7 | Oral, a single dose | 1.5 | 3 |
| G6 | Comparative group | 3 | Control Drug 1 (Vonoprazan) | Oral, a single dose | 0.1 | 0.2 |

### Measurement of pharmacokinetic profiles

Blood samples were obtained from peripheral veins at 30 minutes, 1 hour, 2.5 hours, 4.5 hours, 7.5 hours, 25.5 hours, and 49.5 hours after drug administration, respectively. Blood samples were centrifuged (2 to 8°C, 3,200 g, 10 minutes) to obtain plasma. The amount of the administered compound into the plasma samples was quantified by using LC-MS/MS. Pharmacokinetic parameters were obtained by using a non-compartmental model in Phoenix WinNonlin 6.3 software. 'AUC₀₋ₗₐₛₜ' indicates the area under the time curve, 'C ₘₐₓ' indicates the highest plasma concentration, ' Tₘₐₓ' indicates the time to reach the highest plasma concentration, and 'T_{1/2}' indicates the half-life. The results are shown in Table 5 below.

**[Table 5]**

| **Groups** | **(n=3)** | **AUCₗₐₛₜ (ng.h/mL)** | **Cₘₐₓ (ng/mL)** | **Tₘₐₓ (h)** | **T_{1/2} (h)** |
|---|---|---|---|---|---|
| Vehicle group | mean | - | - | - | - |
| | SD | - | - | - | - |
| Administration group 1 | mean | 99.4 | 32.3 | 0.833 | 1.67 |
| | SD | 20.7 | 3.14 | 0.289 | 0.244 |
| Administration group 2 | mean | 808 | 165 | 1.0 | 2.93 |
| | SD | 274 | 75.8 | 0.0 | 0.883 |
| Administration group 3 | mean | 2894 | 397 | 1.0 | 3.03 |
| | SD | 2061 | 186 | 0.0 | 0.307 |
| Administration group 4 | mean | 19153 | 1430 | 2.17 | 4.29 |
| | SD | 7707 | 261 | 2.02 | 0.805 |
| Comparative Group | mean | 85.5 | 36.4 | 1.0 | 1.29 |
| | SD | 47.5 | 20.0 | 0 | 0.10 |

As shown in Table 5 above, the group administered with Example 7 of the present invention showed a higher area under the time curve and half-life even at a lower dose (0.1 mg/kg), as compared to the group (0.2 mg/kg) administered with Control Drug 1 compound (vonoprazan), a representative P-CAB commercial formulation, and showed peak plasma concentration and time to reach peak plasma concentration which were equivalent to vonoprazan, even at a dose twice lower. In other words, it was confirmed that the pharmacokinetic profile of the compound was greatly improved. In particular, Test Groups 3 and 4 showed a half-life three times longer than that of vonoprazan.

### Measurement of pharmacodynamic profile (pH of Gastric Juice)

To measure the neutralizing power of gastric acid for drug efficacy evaluation, each dose of the compound was administered to each group. Then, histamine dihydrochloride at 0.03 mg/kg was administered subcutaneously 1, 3, 6, 24, and 48 hours after compound administration. The pH of the gastric juice was measured 0, 2.5, 4.5, 7.5, 25.5, and 49.5 hours after compound administration. The results are shown in Table 6 below.

**[Table 6]**

| **Groups** | **(n=3)** | **Average pH** | | | | | |
|---|---|---|---|---|---|---|---|
| | | **0 h** | **2.5 h** | **4.5 h** | **7.5 h** | **25.5 h** | **49.5 h** |
| Vehicle group | mean | 7.0 | 1.9 | 4.0 | 3.4 | 2.7 | 2.6 |
| | SD | 0.0 | 0.1 | 0.4 | 0.5 | 0.3 | 0.4 |
| Administration Group 1 | mean | 6.8 | 3.3 | 6.5 | 6.0 | 2.7 | 2.4 |
| | SD | 1.0 | 1.1 | 0.5 | 1.7 | 0.3 | 0.1 |
| Administration Group 2 | mean | 7.2 | 6.7 | 7.0 | 6.5 | 2.3 | 2.8 |
| | SD | 0.8 | 0.6 | 0.0 | 0.5 | 0.4 | 0.8 |
| Administration Group 3 | mean | 5.7 | 7.2 | 6.7 | 6.2 | 3.0 | 2.3 |
| | SD | 2.3 | 0.3 | 0.3 | 0.3 | 1.3 | 0.3 |
| Administration Group 4 | mean | 6.0 | 7.3 | 7.0 | 6.3 | 6.2 | 3.0 |
| | SD | 1.7 | 0.3 | 0.0 | 0.3 | 0.8 | 1.3 |
| Comparative Group | mean | 6.8 | 4.8 | 6.3 | 4.8 | 2.3 | 3.0 |
| | SD | 0.3 | 2.5 | 0.3 | 1.6 | 0.5 | 0.3 |

As shown in Table 6 above, it was confirmed that the group administered with Example 7 of the present invention showed an excellent gastric acid neutralization effect, similar to the group administered with Control Drug 1 compound (vonoprazan).

### Test Example 5. Evaluation of oral bioavailability in beagle dogs

### Animal models

To evaluate oral bioavailability, Example 7 was administered intravenously or orally to beagle dogs. The classification of the test groups and the dosage of the administered substances are shown in Table 7 below.

**[Table 7]**

| **Groups** | | **Number of animals** | **Administer ed substance** | **Route of admin.** | **Concentration of samples (mg/ml)** | **Dosag e (mg/k g)** | **Number of admin.** |
|---|---|---|---|---|---|---|---|
| G1 | Administration Group 1 | 3 | Example 7 | Intraveno us | 0.1 | 0.2 | Once/ day |
| G2 | Administration Group 2 | 3 | Example 7 | Oral | 1 | 2 | Once/ day |
| G3 | Administration Group 3 | 3 | Example 7 | Oral | 3 | 6 | Once/ day |
| G4 | Administration | 3 | Example 7 | Oral | 10 | 20 | Once/ |
| | Group 4 | | | | | | day |

### Evaluation of bioavailability

For the intravenous administration group, blood samples were obtained from peripheral veins 1 minute, 5 minutes, 15 minutes, 30 minutes, 1 hour, 2 hours, 4 hours, 8 hours, and 24 hours after drug administration. For the oral administration group, blood samples were obtained from peripheral veins 15 minutes, 30 minutes, 1 hour, 2 hours, 4 hours, 6 hours, 8 hours, 12 hours, and 24 hours after drug administration. Blood samples collected at different times were treated with EDTA and centrifuged (2 to 8°C, 3,200 g, 10 minutes) to separate plasma.

The amount of administered compound into the plasma sample was quantified by using LC-MS/MS. Pharmacokinetic parameters were obtained by using a non-compartmental model in Phoenix WinNonlin 6.3 software.

'AUC₀₋ₗₐₛₜ' indicates the area under the time curve, ' Cₘₐₓ' indicates the highest plasma concentration, 'Tₘₐₓ' indicates the time to reach the highest plasma concentration, 'T_{1/2}' indicates the half-life, and 'BA' indicates the bioavailability. The results are shown in Table 8 below.

**[Table 8]**

| **Groups** | **(n=3)** | **AUCₗₐₛₜ(ng.h/ml)** | **Cₘₐₓ(ng/ml)** | **Tₘₐₓ (h)** | **T_{1/2} (h)** | **BA (%)** |
|---|---|---|---|---|---|---|
| Administration Group 1 | mean | 423 | - | - | 3.57 | - |
| | SD | 140 | - | - | 0.711 | |
| Administration Group 2 | mean | 6941 | 632 | 1.53 | 5.55 | >100 |
| | SD | 1256 | 152 | 5.15 | 0.568 | |
| Administration Group 3 | mean | 15141 | 1122 | 3.67 | 7.49 | >100 |
| | SD | 7740 | 269 | 3.79 | 3.35 | |
| Administration Group 4 | mean | 49268 | 3347 | 3.33 | 18.3 | >100 |
| | SD | 17427 | 1175 | 1.15 | 10.7 | |

As shown in Table 8 above, Example 7 showed a bioavailability of at least 100% at concentrations of 1 mg/mL, 3 mg/mL and 10 mg/mL, respectively.

### Test Example 6. Evaluation of acute oral toxicity in rats

An attempt was made to evaluate the acute toxicity of the compound of Example 7 on the fumarate form (Example 41). Twenty female rats (Sprague-Dawley Rats) were obtained, acclimatized, and distributed into a total of four groups, five rats per test group. As feed, irradiated solid feed was allowed to be freely consumed, and, as water, tap water disinfected with an ultraviolet sterilizer and micro-filtration device was placed in a polycarbonate drinking bottle and allowed to be freely consumed. Rats were raised in an animal breeding area maintained at a temperature of 23 ± 3 °C, with a relative humidity of 50 ± 20%, a ventilation frequency of 10 to 15 times/hr, for a lighting time of 12 hours (lights on at 8 a.m. to lights off at 8 p.m.), and with an illuminance of 150 to 300 Lux. As the administration, oral administration, the clinically planned route, was selected for evaluation, and, as the administration dose, the highest dose was set at 600 mg/kg, referring to the results of vonoprazan. The conditions for administration are shown in Table 9 below.

**[Table 9]**

| **Group** | **Concentration of admin. (mg/kg)** | **Administered substance** | **Number of animals** | **Gender** | **Route of admin.** | **Number of admin.** | **Survival rate (%)** |
|---|---|---|---|---|---|---|---|
| G1 | 0 | Example 41 | 5 | Female | Oral | 1 | 100 |
| G2 | 50 | | 5 | | | | 100 |
| G3 | 200 | | 5 | | | | 100 |
| G4 | 400 | | 5 | | | | 100 |
| G5 | 600 | | 4 | | | | 0 |

When administered orally at concentrations of 50 mg/kg, 200 mg/kg, and 400 mg/kg, no deaths were observed, resulting in a survival rate of 100%. At a concentration of 600 mg/kg, all individuals died, resulting in a survival rate of 0%.

### Test Example 7. Evaluation of pharmacokinetics in rats according to deuterium substitution

In order to evaluate the impact on the deuterium substituent in terms of pharmacokinetics, the blood concentrations of Example 35 and Comparative Example 2 in rats were measured and compared. Six male rats (Sprague-Dawley Rats) were obtained, acclimatized, and then distributed into two groups with three rats per test group. As feed, irradiated solid feed was allowed to be freely consumed, and, as water, tap water disinfected with an ultraviolet sterilizer and micro-filtration device was placed in a polycarbonate drinking bottle and allowed to be freely consumed. Rats were raised in an animal breeding area maintained at a temperature of 23 ± 3 °C, with a relative humidity of 50 ± 20%, a ventilation frequency of 10 to 15 times/hr, for a lighting time of 12 hours (lights on at 8 a.m. to lights off at 8 p.m.), and with an illuminance of 150 to 300 Lux. The classification of test groups and the dosage of administered substances are shown in Table 10 below.

**[Table 10]**

| **Groups** | **Concentration of admin. (mg/kg)** | **Administered substance** | **Number of animals** | **Gender** | **Route of admin.** | **Number of admin.** |
|---|---|---|---|---|---|---|
| G1 | 10 | Comparative Example 2 | 3 | Male | Oral | 1 |
| G2 | 10 | Example 35 | 3 | Male | Oral | 1 |

Blood samples were obtained from the jugular vein at 0.5, 1, 2, 5, and 8 hours after drug administration, respectively. Blood samples were centrifuged (2 to 8°C, 3,200 g, 10 minutes) to obtain plasma. The amount of administered compound into the plasma sample was quantified by using LC-MS/MS.

**[Table 11]**

| **Time elapsed after administration** | **Blood concentration (ng/mL)** | |
|---|---|---|
| | **G1** | **G2** |
| 0.5 hr | 59.67 | 301.6 |
| 1 hr | 115.82 | 254.5 |
| 2 hr | 83.81 | 199.2 |
| 5 hr | 12.81 | 22.6 |
| 8 hr | 5.10 | 4.8 |

As shown in Table 11 above, it was confirmed that Example 35, a deuterium-substituent, showed a blood concentration level 5 times higher 0.5 hours after administration, and a blood concentration level at least 2 times higher after 1 hour and 2 hours, as compared to Comparative Example 2.

### Test Example 8. Effect of improving storage stability (related substance identification test) due to deuterium substitution

An attempt was made to evaluate the storage stability over time of the compound of Example 7 of the present invention and the compound of Comparative Example 1 (which is the deuterium-unsubstituted form of the compound of Example 7) depending on the presence of deuterium substitution. The stability of each compound was measured while stored at -2 to 8 °C for 3 months, and the results are shown in Table 12 below.

**[Table 12]**

| Compound | **Initial** | **1^{st} week** | **2^{nd} week** | **3^{rd} week** | **4^{th} week** | **3 months** | **Results** |
|---|---|---|---|---|---|---|---|
| Comparative Example 1 | 97.99 | 90.61 | 81.40 | 76.47 | 70.39 | 53.8 | Unstable (unsuitable) |
| Example 7 | 98.38 | 98.33 | 98.11 | 98.11 | 98.20 | 98.9 | Stable (suitable) |

As shown in Table 12, it was confirmed that the storage stability over time of the compound of Example 7, in which the portion of the methylmethanamine was substituted with deuterium, was significantly improved, as compared to the compound of Comparative Example 1, which was unsubstituted with deuterium.

## Claims

1. A compound represented by the following formula 1: 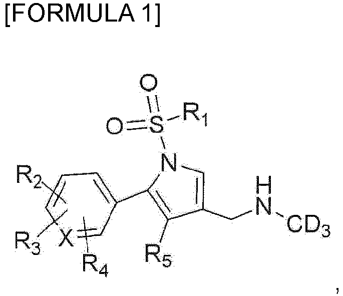 or a pharmaceutically or sitologically acceptable salt thereof,
wherein
R₁ is hydrogen, phenyl, quinolinyl, pyridinyl, pyrimidinyl, piperidinyl, thienyl or imidazolyl, and wherein R₁ except hydrogen is unsubstituted or substituted with 1 to 5 substituents independently selected from the group consisting of C₁-C₆ alkyl, C₁-C₆ alkoxy, C₁-C₄ haloalkyl, C₁-C₄ haloalkoxy, halogen, cyano, nitro, phenyl and halophenyl,
R₂, R₃ and R₄ are each independently hydrogen or halogen, provided that R₂, R₃ and R₄ are not hydrogen at the same time,
R₅ is hydrogen, methoxy, fluoro, chloro or hydroxy, and
X is carbon or nitrogen.

2. The compound or pharmaceutically or sitologically acceptable salt thereof according to claim 1, wherein R₁ is phenyl, thienyl, or imidazolyl, and wherein R₁ is unsubstituted or substituted with 1 to 5 substituents independently selected from the group consisting of C₁-C₆ alkyl, C₁-C₆ alkoxy, C₁-C₄ haloalkyl, C₁-C₄ haloalkoxy, halogen, cyano, nitro, phenyl and halophenyl.

3. The compound or pharmaceutically or sitologically acceptable salt thereof according to claim 1, wherein R₁ is phenyl, quinolinyl, pyridinyl, pyrimidinyl, piperidinyl, thienyl or imidazolyl, and wherein R₁ is unsubstituted or substituted with 1 to 3 substituents independently selected from the group consisting of methyl, trifluoromethyl, methoxy, trifluoromethoxy, fluoro, chloro, bromo, cyano and fluorophenyl.

4. The compound or pharmaceutically or sitologically acceptable salt thereof according to claim 1, wherein R₁ is phenyl, and wherein R₁ is unsubstituted or substituted with 1 or 2 substituents independently selected from the group consisting of methyl, trifluoromethyl, methoxy, trifluoromethoxy, fluoro, chloro, bromo, cyano and fluorophenyl.

5. The compound or pharmaceutically or sitologically acceptable salt thereof according to claim 1, wherein R₁ is thienyl or imidazolyl, and wherein R₁ is unsubstituted or substituted with 1 or 2 substituents independently selected from the group consisting of chloro and methyl.

6. The compound or pharmaceutically or sitologically acceptable salt thereof according to claim 1, wherein R₂, R₃ and R₄ are each independently hydrogen or fluoro, provided that R₂, R₃ and R₄ are not hydrogen at the same time.

7. The compound or pharmaceutically or sitologically acceptable salt thereof according to claim 1, wherein the compound is represented by the following formula 1-1: 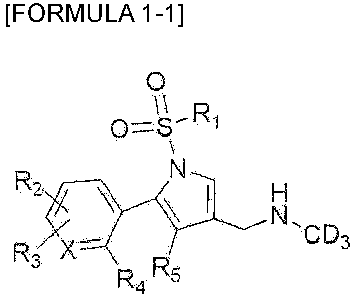 wherein R₁ to R₅ are as defined in claim 1.

8. The compound or pharmaceutically or sitologically acceptable salt thereof according to claim 7, wherein R₂ and R₃ are each independently hydrogen or fluoro, and
R₄ is fluoro.

9. The compound or pharmaceutically or sitologically acceptable salt thereof according to claim 1,
wherein the compound is selected from the group consisting of the following compounds:
1) N-((5-2-fluorophenyl)-1-((4-(trifluoromethyl)phenyl)sulfonyl)-1H-pyrrol-3-yl)methyl)methan-d₃-amine;
2) N-((5-(2-fluorophenyl)-1-tosyl-1H-pyrrol-3-yl)methyl)methan-d₃-amine;
3) N-((5-(2-fluorophenyl)-1-((4-methoxyphenyl)sulfonyl)-1H-pyrrol-3-yl)methyl)methan-d₃-amine;
4) 4-((2-(2-fluorophenyl)-4-(((methyl-d₃)amino)methyl)-1H-pyrrol-1-yl)sulfonyl)benzonitrile;
5) N-((1-((4-bromophenyl)sulfonyl)-5-(2-fluorophenyl)-1H-pyrrol-3-yl)methyl)methan-d₃-amine;
6) N-((1-((4-chlorophenyl)sulfonyl)-5-(2-fluorophenyl)-1H-pyrrol-3-yl)methyl)methan-d₃-amine;
7) N-((5-(2-fluorophenyl)-1-((4-fluorophenyl)sulfonyl)-1H-pyrrol-3-yl)methyl)methan-d₃-amine;
8) N-((5-(2-fluorophenyl)-1-((4-(trifluoromethoxy)phenyl)sulfonyl)-1H-pyrrol-3-yl)methyl)methan-d₃-amine;
9) N-((1-((4'-fluoro-[1,1'-biphenyl]-4-yl)sulfonyl)-5-(2-fluorophenyl)-1H-pyrrol-3-yl)methyl)methan-d₃-amine;
10) N-((1-((4-chlorophenyl)sulfonyl)-5-(3-fluorophenyl)-1H-pyrrol-3-yl)methyl)methan-d₃-amine;
11) N-((5-(3-fluorophenyl)-1-((4-methoxyphenyl)sulfonyl)-1H-pyrrol-3-yl)methyl)methan-d₃-amine;
12) N-((5-(2,4-difluorophenyl)-1-((4-fluorophenyl)sulfonyl)-1H-pyrrol-3-yl)methyl)methan-d₃-amine;
13) N-((1-((4-bromophenyl)sulfonyl)-5-(2,4-difluorophenyl)-1H-pyrrol-3-yl)methyl)methan-d₃-amine;
14) N-((5-(2,4-difluorophenyl)-1-((4-(trifluoromethyl)phenyl)sulfonyl)-1H-pyrrol-3-yl)methyl)methan-d₃-amine;
15) N-((5-(2,4-difluorophenyl)-1-((4-(trifluoromethoxy)phenyl)sulfonyl)-1H-pyrrol-3-yl)methyl)methan-d₃-amine;
16) N-((5-(2,4-difluorophenyl)-1-tosyl-1H-pyrrol-3-yl)methyl)methan-d₃-amine;
17) N-((5-(2,4-difluorophenyl)-1-((4-methoxyphenyl)sulfonyl)-1H-pyrrol-3-yl)methyl)methan-d₃-amine;
18) 4-((2-(2,4-difluorophenyl-4-(((methyl-d₃)amino)methyl)-1H-pyrrol-1-yl)sulfonyl)benzonitrile;
19) N-((5-(3-fluorophenyl)-1-((4-fluorophenyl)sulfonyl)-1H-pyrrol-3-yl)methyl)methan-d₃-amine;
20) N-((1-((4-bromophenyl)sulfonyl)-5-(3-fluorophenyl)-1H-pyrrol-3-yl)methyl)methan-d₃-amine;
21) N-((5-(3-fluorophenyl)-1-tosyl-1H-pyrrol-3-yl)methyl)methan-d₃-amine;
22) 4-((2-(3-fluorophenyl)-4-(((methyl-d₃)amino)methyl)-1H-pyrrol-1-yl)sulfonyl)benzonitrile;
23) N-((5-(3-fluorophenyl)-1-((4-(trifluoromethyl)phenyl)sulfonyl)-1H-pyrrol-3-yl)methyl)methan-d₃-amine;
24) N-((5-(4-fluorophenyl)-1-((4-fluorophenyl)sulfonyl)-1H-pyrrol-3-yl)methyl)methan-d₃-amine;
25) N-((1-((4-bromophenyl)sulfonyl)-5-(4-fluorophenyl)-1H-pyrrol-3-yl)methyl)methan-d₃-amine;
26) N-((1-((4-chlorophenyl)sulfonyl)-5-(4-fluorophenyl)-1H-pyrrol-3-yl)methyl)methan-d₃-amine;
27) N-((5-(4-fluorophenyl)-1-((4-methoxyphenyl)sulfonyl)-1H-pyrrol-3-yl)methyl)methan-d₃-amine;
28) N-((5-(4-fluorophenyl)-1-tosyl-1H-pyrrol-3-yl)methyl)methan-d₃-amine;
29) N-((5-(4-fluorophenyl)-1-((4-(trifluoromethoxy)phenyl)sulfonyl)-1H-pyrrol-3-yl)methyl)methan-d₃-amine;
30) N-((5-(4-fluorophenyl)-1-((4-(trifluoromethyl)phenyl)sulfonyl)-1H-pyrrol-3-yl)methyl)methan-d₃-amine;
31) 4-((2-(4-fluorophenyl)-4-(((methyl-d₃)amino)methyl)-1H-pyrrol-1-yl)sulfonyl)benzonitrile;
32) N-((5-(3-fluorophenyl)-1-((4-(trifluoromethoxy)phenyl)sulfonyl)-1H-pyrrol-3-yl)methyl)methan-d₃-amine;
33) N-((1-((4-fluorophenyl)sulfonyl)-5-(2-fluoropyridin-3-yl)-1H-pyrrol-3-yl)methyl)methan-d₃-amine;
34) N-((1-((4-chlorophenyl)sulfonyl)-5-(2-fluoropyridin-3-yl)-1H-pyrrol-3-yl)methyl)methan-d₃-amine;
35) N-((5-(2,4-difluorophenyl)-4-methoxy-1-(pyridin-3-ylsulfonyl)-1H-pyrrol-3-yl)methyl)methan-d₃-amine;
36) N-((5-(2-fluorophenyl)-4-methoxy-1-(pyridin-3-ylsulfonyl)-1H-pyrrol-3-yl)methyl)methan-d₃-amine;
37) N-((5-(2,4-difluorophenyl)-4-methoxy-1-((6-methoxypyridin-3-yl)sulfonyl)-1H-pyrrol-3-yl)methyl)methand₃-amine;
38) N-((4-methoxy-1-((6-(trifluoromethyl)pyridin-3-yl)sulfonyl)-5-(2,4,6-trifluorophenyl)-1H-pyrrol-3-yl)methyl)methan-d₃-amine;
39) N-((4-methoxy-1-(pyridin-3-ylsulfonyl)-5-(2,4,6-trifluorophenyl)-1H-pyrrol-3-yl)methyl)methan-d₃-amine; and
40) N-((4-methoxy-1-((6-methoxypyridin-3-yl)sulfonyl)-5-(2,4,6-trifluorophenyl)-1H-pyrrol-3-yl)methyl)methan-d₃-amine.

10. A pharmaceutical composition for preventing or treating gastrointestinal diseases, comprising the compound or pharmaceutically acceptable salt thereof according to any one of claims 1 to 9.

11. The pharmaceutical composition for preventing or treating gastrointestinal diseases according to claim 10, wherein the gastrointestinal diseases are one or more types selected from gastric ulcer, duodenal ulcer, gastritis, reflux esophagitis, gastric mucosal damage and stomach cancer.

12. A health functional food composition for preventing or improving gastrointestinal diseases, comprising the compound or sitologically acceptable salt thereof according to any one of claims 1 to 9.

13. The health functional food composition for preventing or improving gastrointestinal diseases according to claim 12, wherein the gastrointestinal diseases are one or more types selected from gastric ulcer, duodenal ulcer, gastritis, reflux esophagitis, gastric mucosal damage and stomach cancer.

14. A method for producing a pyrrole derivative comprising the steps of:
(1) reacting a compound of the following formula 2 and a sulfonyl compound (R₁SO₂Cl) in the presence of an organic solvent and a base to prepare a compound of the following formula 3; and
(2) reacting the compound of the following formula 3 and deuterated methylamine (methyl-d₃-amine) in the presence of an organic solvent and a reducing agent to prepare a compound of the following formula 4: 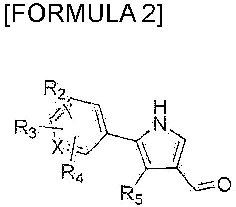 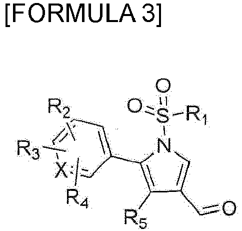 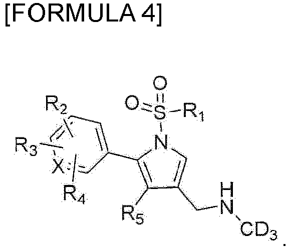 wherein,
R₁ is hydrogen, phenyl, quinolinyl, pyridinyl, pyrimidinyl, piperidinyl, thienyl or imidazolyl, and wherein R₁ except hydrogen is unsubstituted or substituted with 1 to 5 substituents independently selected from the group consisting of C₁-C₆ alkyl, C₁-C₆ alkoxy, C₁-C₄ haloalkyl, C₁-C₄ haloalkoxy, halogen, cyano, nitro, phenyl and halophenyl,
R₂, R₃ and R₄ are each independently hydrogen or fluoro, provided that R₂, R₃ and R₄ are not hydrogen at the same time,
R₅ is hydrogen or chloro, and
X is carbon or nitrogen.

15. A method for producing the pyrrole derivative of formula 13, comprising the steps:
(1) reacting the compound of the following formula 9 and a sulfonyl compound (R₁SO₂Cl) in the presence of a chloroform organic solvent and a base to prepare a compound of the following formula 10;
(2) reacting the compound of the following formula 10 and a reducing agent in the presence of an organic solvent to prepare a compound of the following formula 11;
(3) reacting the compound of the following formula 11 in the presence of an oxidizing agent to prepare a compound of the following formula 12; and
(4) reacting the compound of the following formula 12 and deuterated methylamine (methyl-d₃-amine) in the presence of an organic solvent and a reducing agent to prepare a compound of the following formula 13: 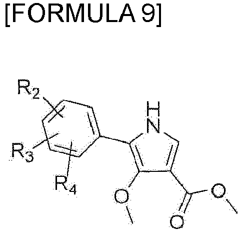 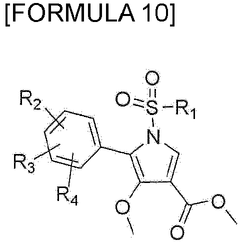 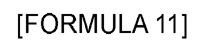 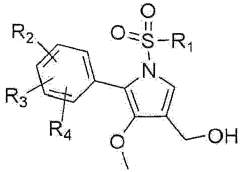 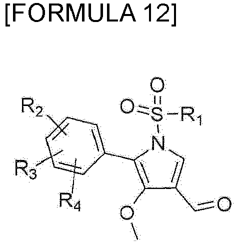 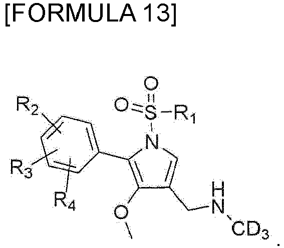 wherein,
R₁ is hydrogen, phenyl, quinolinyl, pyridinyl, pyrimidinyl, piperidinyl, thienyl or imidazolyl, and wherein R₁ except hydrogen is unsubstituted or substituted with 1 to 5 substituents independently selected from the group consisting of C₁-C₆ alkyl, C₁-C₆ alkoxy, C₁-C₄ haloalkyl, C₁-C₄ haloalkoxy, halogen, cyano, nitro, phenyl and halophenyl, and
R₂, R₃ and R₄ are each independently hydrogen or fluoro, provided that R₂, R₃ and R₄ are not hydrogen at the same time.
